(19) Europäisches Patentamt
European Patent Office
Office européen des brevets

(11) EP 0 582 503 B1

(12) FASCICULE DE BREVET EUROPEEN

(45) Date de publication et mention de la délivrance du brevet: **27.11.1996 Bulletin 1996/48**

(51) Int. Cl.$^6$: **A61K 7/00**, A61K 9/127

(21) Numéro de dépôt: **93401902.7**

(22) Date de dépôt: **22.07.1993**

# (54) Composition formée d'une dispersion aqueuse de vésicules de lipides amphiphiles non-ioniques stabilisées

Aus einer wässrigen Dispersion von stabilisierten Vesikeln aus amphiphilen nichtionischen Lipiden gebildetes Mittel

Composition formed of an aqueous dispersion of stabilized vesicles of amphiphilic non-ionic lipids

(84) Etats contractants désignés:
**AT BE CH DE DK ES FR GB GR IE IT LI NL PT SE**

(30) Priorité: **03.08.1992 FR 9209603**
**15.10.1992 FR 9212343**

(43) Date de publication de la demande:
**09.02.1994 Bulletin 1994/06**

(73) Titulaire: **L'OREAL**
**75008 Paris (FR)**

(72) Inventeurs:
- **Ribier, Alain**
**F-75001 Paris (FR)**
- **Simonnet, Jean-Thierry**
**F-75011 Paris (FR)**
- **Handjani, Rose-Marie**
**F-75014 Paris (FR)**
- **Terren, Nadia**
**F-94550 Chevilly-Larue (FR)**

(74) Mandataire: **Peuscet, Jacques et al**
**SCP Cabinet Peuscet et Autres,**
**68, rue d'Hauteville**
**75010 Paris (FR)**

(56) Documents cités:
**EP-A- 0 319 638**
**EP-A- 0 433 131**
**WO-A-90/06747**
**FR-A- 2 552 666**
**US-A- 4 670 185**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 582 503 B1

Printed by Rank Xerox (UK) Business Services
2.13.8/3.4

## Description

La présente invention concerne une composition formée d'une dispersion aqueuse de vésicules de certains lipides amphiphiles non-ioniques, qui sont stabilisées et ne se dégradent pas au cours du stockage. Cette composition est particulièrement intéressante en cosmétique et en dermopharmacie.

On sait que certains lipides amphiphiles possèdent la propriété de former des phases mésomorphes, dont l'état d'organisation est intermédiaire entre l'état cristallin et l'état liquide et que, parmi eux, certains sont susceptibles de gonfler en présence d'une solution aqueuse pour former une phase lamellaire puis, après agitation, former des vésicules ou sphérules dispersées dans une phase aqueuse. Ces vésicules sont formées par une membrane constituée par des feuillets sensiblement concentriques comportant une ou plusieurs couches multimoléculaires, de préférence bimoléculaires, encapsulant une phase aqueuse.

Les vésicules susmentionnées peuvent être préparées par de nombreux procédés. Selon un premier procédé, qui est par exemple décrit par BANGHAM et al. (J. Mol. Bio., 13, 1965 - pages 238 à 262), on dissout la phase lipidique dans un solvant volatil, on forme un film mince de phase lipidique sur les parois d'un flacon par évaporation du solvant, on introduit la phase à encapsuler sur le film lipidique et on agite le mélange mécaniquement jusqu'à obtention de la dispersion de vésicules à la taille désirée ; on obtient ainsi une dispersion aqueuse de vésicules encapsulant une phase aqueuse, la phase aqueuse encapsulée et la phase aqueuse de dispersion étant identiques. Selon un second procédé dit "par cofusion des lipides", décrit par exemple dans FR-A-2 315 991, on prépare la phase lipidique par mélange du (des) lipide(s) amphiphile(s) et des éventuels additifs, à une température où le mélange est fondu, si le mélange n'est pas liquide à température ambiante ; on forme une phase lamellaire, par introduction de la phase aqueuse à encapsuler ; puis on disperse la phase lamellaire sous forme de vésicules, à l'aide d'un ultra-disperseur, d'un homogénéiseur ou d'ultrasons, dans une phase aqueuse de dispersion. Dans une variante de ce procédé, la formation de la phase lamellaire ne constitue pas un stade séparé du procédé. Les vésicules obtenues par ces deux procédés sont généralement de type "multifeuillet". Pour obtenir des vésicules de type "monofeuillet", on peut utiliser l'enseignement de FR-A-2 543 018.

Quel que soit le procédé utilisé, les vésicules sont obtenues sous forme de dispersion dans une phase aqueuse.

De façon connue, les vésicules de lipides amphiphiles peuvent contenir des actifs cosmétiques ou pharmaceutiques soit dans la phase aqueuse encapsulée si lesdits actifs sont hydrosolubles, soit dans la membrane lipidique s'ils sont oléosolubles. Des actifs peuvent également être présents dans la phase aqueuse de dispersion.

Les lipides amphiphiles utilisés pour l'obtention des vésicules sont des lipides ayant pour formule générale :

$$X - Y$$

formule dans laquelle X représente un groupe hydrophile et Y représente un groupe lipophile. Les lipides amphiphiles peuvent être des lipides ioniques, pour lesquels le groupe X est ionique, ou des lipides non-ioniques pour lesquelles le groupe X est non-ionique.

De façon connue, on peut utiliser pour la fabrication des vésicules des mélanges de lipides amphiphiles ioniques, des mélanges de lipides amphiphiles non-ioniques et des mélanges de ces deux types de lipides.

On a proposé, par exemple dans FR-A 2 315 991 et EP-A-0 433 131, de préparer des vésicules à partir de lipides amphiphiles non-ioniques constituées par des esters de polyols, oxyéthylénés ou non. Les vésicules préparées avec lesdits esters sont intéressantes en cosmétique et dermopharmacie car elles sont biodégradables dans et sur la peau. Mais elles présentent un inconvénient majeur : en présence d'eau, on constate une hydrolyse des groupements esters dans la membrane lipidique, ce phénomène d'hydrolyse étant particulièrement important dès que l'on s'écarte d'un pH proche de la neutralité. Pour remédier à cet inconvénient, on a proposé d'introduire dans la phase aqueuse de dispersion des vésicules une solution tampon saline maintenant le pH à une valeur comprise entre 6 et 7, de préférence voisine de 6,5. Mais l'introduction de tampons salins dans les compositions utilisées en cosmétique ou en dermopharmacie présente de nombreux inconvénients. Les tampons salins sont incompatibles avec de nombreux additifs ou actifs couramment utilisés en cosmétique, notamment, ils abaissent la viscosité des compositions contenant un gélifiant anionique tel que les mélanges carboxyvinyliques commercialisés sous la dénomination "CARBOPOL" par la société "GOODRICH" ; ils provoquent aussi la précipitation des protéines. La présence d'un tampon salin réduit la charge électrique (potentiel zéta) des vésicules, ce qui entraîne leur floculation. De plus, la présence dudit tampon salin provoque une forte corrosion des métaux, en particulier des aciers, constituant les surfaces des homogénéiseurs utilisés pour la fabrication des vésicules. Enfin, les compositions cosmétiques contenant un tampon salin confèrent, après application sur la peau, un toucher plus rèche.

Selon la présente invention, on a trouvé un moyen permettant d'obtenir avec certains esters de polyol, des dispersions dans lesquelles les vésicules sont stables dans l'eau ; ces dispersions peuvent être, par conséquent, conservées et utilisées pour la préparation de compositions cosmétiques ou dermopharmaceutiques, sans qu'il soit nécessaire d'introduire un tampon salin.

Selon la présente demande, on a trouvé qu'en associant dans la phase lipidique constituant la membrane des vésicules certains esters de polyol avec au moins un lipide ionique pris dans un certain groupe, on obtenait des vésicules stables à l'hydrolyse.

La présente invention a, par conséquent, pour objet une composition formée d'une dispersion aqueuse de vésicules constituées par une membrane de phase lipidique encapsulant une phase aqueuse, la phase lipidique comprenant des lipides amphiphiles non ioniques et au moins un lipide amphiphile ionique, caractérisée par le fait que :

- les lipides amphiphiles non-ioniques sont constitués par un mélange d'esters d'au moins un polyol choisi dans le groupe formé par le polyéthylèneglycol comportant de 1 à 60 unités oxyde d'éthylène, le sorbitane, le sorbitane portant 2 à 60 unités d'oxyde d'éthylène, le glycérol portant 2 à 30 unités d'oxyde d'éthylène, les polyglycérols comportant 2 à 15 unités glycérol, les sucroses, les glucoses portant 2 à 30 unités oxyde d'éthylène, et d'au moins un acide gras comportant une chaîne alkyle en $C_5$-$C_{17}$, saturée ou non saturée, linéaire ou ramifiée, le nombre de chaînes alkyle par groupe polyol étant compris entre 1 et 10 ; et que
- le(s) lipide(s) amphiphile(s) ionique(s) est(sont) choisi(s) dans le groupe formé par ceux conférant à la dispersion un pH compris entre 5,5 et 7,5 ;
- le rapport en poids entre la quantité de lipides amphiphiles nonioniques et celle de lipide(s) amphiphile(s) ionique(s) dans la phase lipidique étant compris entre 50/1 et 50/25 et le rapport en poids entre la phase lipidique et la phase aqueuse de dispersion étant compris entre 1/1 000 et 300/1 000.

On a constaté que, dans la composition selon l'invention, les vésicules obtenues étaient stables dans l'eau, en l'absence de solutions salines tamponnées, après conservation pendant 2 mois à des températures comprises entre la température ambiante et 45°C ; elles ont, par conséquent, une stabilité suffisante pour pouvoir être utilisées dans des compositions cosmétiques et dermopharmaceutiques.

La composition selon l'invention permet donc de bénéficier de l'avantage des esters de polyol(s), qui est d'être dégradable sous l'action du pH de la peau ou par les enzymes de la peau. Leur dégradation donne naissance à des produits qui, dans la plupart des cas, ont une action cosmétique ou dermopharmaceutique sur la peau. En effet, les acides gras libres, tels que l'acide palmitique ou l'acide stéarique, ont une activité bactériostatique et les polyols tels que le glycérol, les polyglycérols, le sorbitol ou le sucrose sont des humectants et peuvent éventuellement fournir un apport d'énergie.

Les vésicules obtenues présentent un bon taux d'encapsulation de substances hydrosolubles et une faible perméabilité. Elles permettent donc l'encapsulation d'actifs hydrosolubles. De plus, la dégradation progressive des vésicules sur la peau permet une libération contrôlée des actifs hydrosolubles éventuellement contenus dans la phase aqueuse encapsulée ou des actifs liposolubles éventuellement contenus dans la phase lipidique.

Les dispersions de vésicules de la composition selon l'invention peuvent être préparées par tout procédé connu de fabrication de vésicules de lipides amphiphiles, plus particulièrement par le procédé dit "par cofusion des lipides", ce qui permet de les préparer de façon simple à l'échelle industrielle.

Dans la présente demande et les revendications, l'expression "mélange d'esters" couvre non seulement des mélanges d'esters purs de familles chimiques différentes mais couvre également tout produit, qui contient plusieurs esters de polyol chimiquement purs de la même famille dans des proportions variables. C'est, en particulier, le cas des produits ayant une formule statistique, dans leur partie hydrophile, par exemple un ester de polyglycérol de formule

$$R\ CO\left[OCH_2-CHOH-CH_2\right]_{\bar{n}}OH$$

où $\bar{n}$ est une valeur statistique et qui peut contenir des proportions diverses d'esters pour lesquels n = 1, n = 2, n = 3, n = 4, etc ... ; c'est aussi le cas des esters comportant plusieurs chaînes alkyle dans leur partie lipophile, tels que les cocoates, qui contiennent des chaînes alkyle en $C_5$ à $C_{17}$ ou les isostéarates, où les chaînes alkyle en $C_{17}$ sont un mélange complexe de formes isomères ; c'est également le cas des produits constitués par des mélanges de mono-, di-, tri-ou polyesters d'un même polyol. Il faut noter qu'un produit, qui ne contiendrait qu'un seul ester susceptible de former des vésicules et des impuretés d'un autre type ne pourrait pas être utilisé selon l'invention.

Des esters commerciaux utilisables seuls selon l'invention, car ils sont en réalité des mélanges d'esters, sont par exemple les suivants :

- les esters partiels de sorbitane (ou anhydride de sorbitol) et d'acide gras, vendus sous les dénominations commerciales "SPAN 20, 40, 60 et 80" par la société "ICI" ;
- l'isostéarate de sorbitane, vendu sous la dénomination commerciale "SI 10 R NIKKOL" par la société "NIKKO" ;

- le stéarate de sorbitane portant 4 unités oxyde d'éthylène, vendu sous la dénomination "TWEEN 61" par la société "ICI" ;
- le stéarate de polyéthylène glycol à 8 unités oxyde d'éthylène, vendu sous la dénomination "MYR J 45" par la société "ICI" ;
- le monostéarate du polyéthylène glycol de formule

$$OHCH_2(CH_2OCH_2)_{\overline{n}}CH_2OH$$

formule dans laquelle $\overline{n}$ est égal à 4, vendu sous la dénomination "MYS 4" par la société "NIKKO" ;
- le stéarate de polyéthylène glycol de poids moléculaire 400, qualité chimique ou qualité produite par biotechnologie, vendu par la société "UNICHEMA" ;
- le stéarate de diglycéryle portant 4 unités d'oxyde d'éthylène, vendu sous la dénomination "HOSTACERINE DGS" par la société "HOECHST" ;
- le stéarate de tétraglycérol, vendu sous la dénomination "TETRAGLYN 1S" par la société "NIKKO" ;
- l'isostéarate de diglycéryle, vendu par la société "SOLVAY" ;
- le distéarate de diglycéryle, vendu sous la dénomination "EMALEX DSG 2" par la société "NIHON" ;
- les mono-, di- et tripalmitostéarate de sucrose, vendus sous les dénominations " F50, F70, F110 ET F160 CRODESTA" par la société "CRODA" ;
- le mélange de mono- et dipalmitostéarate de sucrose, vendu sous la dénomination "GRILLOTEN PSE 141 G" par la société "GRILLO" ;
- le mélange de stéarate de sucrose et de cocoate de sucrose, vendu sous la dénomination "ARLATONE 2121" par la société "ICI" ;
- le distéarate de méthylglucose portant 20 unités oxyde d'éthylène, vendu sous la dénomination "GLUCAM E 20 DISTEARATE" par la société "AMERCHOL".

Des mélanges entre eux de ces différents produits, qui sont déjà des mélanges, ou des mélanges de ces produits avec des produits purs peuvent, bien entendu, être utilisés.

Le(s) lipide(s) amphiphile(s) ionique(s) associé(s) aux lipides amphiphiles non-ioniques selon l'invention, est(sont), de préférence, pris dans le groupe formé par :

1)les lipides anioniques neutralisés , ces lipides anioniques étant, de préférence, choisis parmi :

- les sels alcalins du dicétylphosphate, et du dimyristylphosphate en particulier les sels de Na et K ;
- les sels alcalins du cholestérol-sulfate, en particulier le sel de Na ;
- les sels alcalins du cholestérol-phosphate, en particulier le sel de Na ;-les acylglutamates mono- et disodique ;
- le sel de sodium de l'acide phosphatidique ;

2)les lipides amphotères, ces lipides amphotères étant, de préférence, des phospholipides, en particulier la phosphatidyléthanolamine de soja pure ;

3)les dérivés alkylsulfoniques, ces dérivés étant, de préférence, les composés de formule :

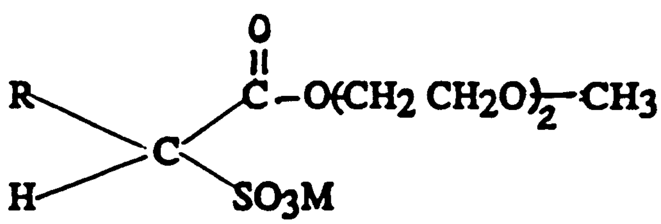

formule dans laquelle R représente les radicaux $C_{16}H_{33}$ et $C_{18}H_{37}$ pris en mélange ou séparément et M est un métal alcalin, de préférence le sodium.

On peut, de façon connue, incorporer dans la phase lipidique constituant la membrane lipidique des vésicules, au moins un additif qui a pour fonction principale de diminuer la perméabilité des vésicules, de prévenir leur floculation et leur fusion et d'augmenter le taux d'encapsulation.

Selon l'invention, on peut ajouter à la phase lipidique au moins un additif choisi, de préférence, dans le groupe formé par :

- les stérols et notamment les phytostérols et le cholestérol,

- les alcools et diols à longue chaîne,
- les amines à longue chaîne et leurs dérivés ammonium quaternaire,

Ces additifs peuvent éventuellement avoir une activité cosmétique et/ou dermopharmaceutique. C'est, par exemple, le cas du cholestérol.

Les vésicules des compositions selon l'invention peuvent contenir, de façon connue, un ou plusieurs composé(s) actif(s) ayant une activité cosmétique et/ou dermopharmaceutique, qui, selon leurs caractéristiques de solubilité, peuvent avoir différentes localisations. Si les actifs sont hydrosolubles, on les introduit dans la phase aqueuse encapsulée des vésicules. Si les actifs sont liposolubles, on les introduit dans la phase lipidique constituant la membrane. Si les actifs sont amphiphiles, ils se répartissent entre la phase lipidique et la phase aqueuse encapsulée avec un coefficient de partage, qui varie selon la nature de l'actif amphiphile et les compositions respectives de la phase lipidique et de la phase aqueuse encapsulée.

Les actifs hydrosolubles sont, par exemple, la glycérine, le sorbitol, l'érythrulose et les antibiotiques. Les actifs liposolubles ou partiellement liposolubles (amphiphiles) sont choisis parmi ceux qui n'augmentent pas de façon significative la perméabilité des vésicules, ne provoquent pas leur floculation et leur fusion et ne diminuent pas le taux d'encapsulation. On utilise avantageusement des actifs liposolubles qui constituent également des additifs .

Les actifs liposolubles préférés selon l'invention sont choisis dans le groupe formé par :

- les sphingomyélines,
- les glycocéramides, en particulier ceux issus de germe de blé et
- les céramides naturels ou de synthèse, de préférence ceux décrits dans la demande de brevet français N°9102091 déposée le 21 Février 1991, ayant pour formule :

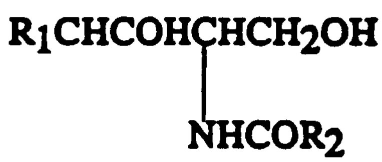

(I)

formule dans laquelle :

$R_1$ représente un radical alkyle ou alcényle en $C_{11}$-$C_{21}$,
$R_2$ représente :

- un radical hydrocarboné en $C_{11}$-$C_{23}$ saturé,
- un mélange de radicaux hydrocarbonés en $C_{11}$-$C_{19}$, linéaires, saturés et portant au moins une insaturation éthylénique, de préférence 1 ou 2, dans lequel la proportion de radicaux saturés ne peut excéder 35%, le céramide de formule (I) étant sous forme d'un mélange racémique de diastéréoisomères érythro et thréo dans des proportions érythro/thréo comprises entre 85/15 et 60/40.

Selon l'invention on introduit, de préférence, dans la phase lipidique constituant la membrane un mélange de céramide(s) et de cholestérol. En effet, l'utilisation de ce mélange est particulièrement intéressante, car elle permet de reconstituer les lipides de la peau, lors de la dégradation des vésicules de la dispersion selon l'invention, sur la peau.

Les actifs introduits, qu'ils soient hydrophiles, lipophiles ou amphiphiles, peuvent avoir des activités cosmétiques et/ou dermopharmaceutiques (ou "fonctions") très variables qui sont données dans le tableau I ci-après :

## TABLEAU I

| FONCTION | ACTIFS UTILISABLES |
|---|---|
| Anti-oxydant ou anti-radicaux libres. | Les extraits des plantes suivantes :<br>- Aubépine.<br>- Ginkgo biloba.<br>- Thé vert.<br>- Vigne.<br>- Romarin<br>Les enzymes :<br>- Commercialisées sous la dénomination SB 12 par SEDERMA et constituées par un mélange de lactoferrine et de lactoperoxydase, de glucose oxydase et de thiocyanate de potassium.<br>- Superoxyde dismutase.<br>- Glutathion peroxydase.<br>La superphycodismutase extraite d'algues.<br>Les coenzymes Q, en particulier la coenzyme Q10.<br>Les séquestrants, en particulier des dérivés d'acides polyphosphoniques.<br>Les tanins.<br>Le sélénium et ses dérivés, en particulier la séléniométhionine.<br>Les peptides, par exemple un mélange d'extraits de rate et de thymus,<br>Thiolim et sérum albumine bovine non stabilisée.<br>Les protéines, par exemple l'hémocyanine qui est une protéine cuivrée extraite de l'escargot marin et l'apohémocyanine qui est une protéine analogue sans cuivre.<br>Les flavonoïdes, notamment la catéchine, les proanthocyanidines, les flavanols, les flavones, les isoflavones, les flavanénols, les flavanones, les flavanes et les chalcones.<br>Les caroténoïdes, notamment le ß-carotène et le rocou.<br>L'acide sorbohydroxyamique.<br>Les tocophérols, notamment l'alpha-tocophérol et l'acétate d'alpha-tocophérol.<br>Le palmitate d'ascorbyle.<br>Le gallate de propyle.<br>L'acide caféique et ses dérivés.<br>L'acide ascorbique.<br>L'acide homogentisique.<br>L'acide érythorbique.<br>L'acide nordihydroguaïacétique.<br>Le laurylméthionate de lysine.<br>Le butylhydroxyanisole.<br>Le butylhydroxytoluène.<br>Les substances "SOD like". |

## TABLEAU I (suite 1)

| FONCTION | ACTIFS UTILISABLES |
|---|---|
| Hydratant ou humectant | Une reconstitution de sueur ("Normal moisturizing factors"-NMF).<br>Le pyroglutamate de sodium.<br>L'acide hyaluronique.<br>Les dérivés de chitosane (carboxyméthylchitine).<br>Le ß-glycérophosphate.<br>Le lactamide.<br>L'acétamide.<br>Le lactate d'éthyle, de sodium et de triéthanolamine.<br>Le pyrrolidone carboxylate de métaux en particulier de Mg, Zn, Fe, Ca ou Na.<br>La thiamorpholinone.<br>L'acide orotique.<br>Les acides carboxyliques alpha-hydroxylés en $C_3$ à $C_{20}$, notamment l'acide alpha-hydroxy propionique.<br>Les polyols, notamment l'inositol, le glycérol, la diglycérine, le sorbitol.<br>Les polyolosides, notamment alginate et guar.<br>Les protéines, notamment le collagène soluble et la gélatine.<br>Les lipoprotides choisis parmi les dérivés mono ou polyacylés d'acides aminés ou de polypeptides dans lesquels le reste acide RCO comporte une chaîne hydrocarbonée en $C_{13}$-$C_{19}$, notamment l'acide palmitoylcaséinique, l'acide palmitoyl collagénique, le dérivé dipalmitoyl-O-N de l'hydroxyproline, le stéaroyl glutamate de sodium, le stéaroyl tripeptide de collagène, l'oléyltétra et pentapeptide de collagène, le linoléate d'hydroxyproline.<br>L'urée et ses dérivés, notamment la méthylurée.<br>L'extrait de tissu cutané, notamment celui commercialisé sous la dénomination "OSMODYN" par les Laboratoires Serobiologiques de Nancy (LSN) et contenant des peptides, des acides aminés, des saccharides et 17% de mannitol.<br>Plus particulièrement une association de glycérol, d'urée et d'acide palmitoylcaséinique. |
| Mélanorégulateur :<br>1) accélérateur de bronzage | Les huiles de bergamote et de citrus.<br>L'alpha-MSH et ses homologues synthétiques.<br>La caféine.<br>Les dérivés de tyrosine, notamment le tyrosinate de glucose et la N-malyltyrosine. |

TABLEAU I (suite 2)

| FONCTION | ACTIFS UTILISABLES |
|---|---|
| 2) Dépigmentant | L'acide ascorbique ou vitamine C et ses dérivés, notamment l'ascorbyle phosphate de Mg.<br>Les hydroxyacides, notamment l'acide glycolique.<br>L'acide kojique.<br>L'arbutine et ses dérivés.<br>L'hémocyanine (protéine cuivrée de l'escargot marin) et l'apohémocyanine (protéine analogue à la précédente sans cuivre).<br>L'hydroquinone et ses dérivés, notamment le monoalkyl éther et le benzyléther. |
| Coloration de la peau (brunissage artificiel) | L'ortho diacétylbenzène.<br>Les indoles.<br>La dihydroxyacétone.<br>L'érythrulose.<br>Le glycéraldéhyde.<br>Les gamma-dialdéhydes, notamment l'aldéhyde tartrique. |
| Liporégulateurs (amincissant et anti-acné, anti-séborrhée) | Les complexes de vitamines et d'oligo-éléments, notamment le complexe vitamine $B_6$/zinc.<br>L'orizanol.<br>L'acide azélaïque.<br>Les xanthines et alkylxanthines, notamment l'extrait de cola, la caféine et la théophylline.<br>L'adénosine monophosphate cyclique et non cyclique.<br>L'adénosine triphosphate.<br>L'extrait de lierre.<br>L'extrait de marron d'Inde.<br>Les extraits d'algues, notamment l'extrait d'algues rouges (fucus serratus) et le cytofiltrat.<br>L'extrait de ginseng.<br>L'extrait de Centella Asiatica (asiaticoside) contenant de la génine et de l'acide asiatique.<br>La thioxolone (HBT).<br>La S-carboxyméthylcystéine.<br>La S-benzylcystéamine. |

TABLEAU I (suite 3)

| FONCTION | ACTIFS UTILISABLES |
|---|---|
| Antivieillissement et anti-rides | Les insaponifiables par exemple de soja et d'avocats.<br>Les acides gras insaturés, notamment l'acide linoléique et l'acide linolénique.<br>Les hydroxyacides, notamment l'acide glycolique.<br>Les facteurs de croissance.<br>Les complexes oligoéléments - vitamines, notamment $B_6$-Zn.<br>L'acide n-octanoyle-5 salicylique.<br>L'adénosine.<br>Le rétinol et ses dérivés, notamment l'acétate de rétinol et le palmitate de rétinol.<br>Les rétinoïdes, notamment les acides rétinoïques cis ou trans et ceux décrits dans les brevets FR-A-2 570 377 ; EP-A-199636 ; et EP-A- 325540 et la demande de brevet européen 90-402072.<br>L'association de rétinoïdes et de xanthines.<br>L'hydroxyproline.<br>Les acides sialiques.<br>L'extrait de rate, de thymus, Thiolim et sérum albumine bovine non stabilisé vendu sous la dénomination commerciale "SILAB" par la Société "SILAB".<br>Un extrait animal placentaire, notamment l'extrait embryonnaire placentaire de bovidés dans l'eau à 5,5 % stabilisé par 0,2 % d'exyl K100a (matrix).<br>Les protéoglycannes, en particulier le protéoglycanne de cartilage de trachée de bovidés à 5 % stabilisé (protéodermin).<br>Le colostrum.<br>Les facteurs d'oxygénation cellulaire, notamment l'octacosamol. |
| Anti-UV | Les filtres UV, notamment le paraméthoxycinnamate d'éthyl-2 hexyle ;<br>la benzophénone,<br>le benzylidène-camphre et leurs dérivés, en particulier, la tétrahydroxy-2,2', 4,4'-benzophénone et l'acide hydroxy-2 méthoxy-4 benzophénone-5 sulfonique ;<br>l'acide paraaminobenzoïque,<br>le salicylate de dipropylèneglycol,<br>l'octyl salicylate,<br>les dérivés de dibenzoylméthane vendus sous les marques EUSOLEX 8020 ou PARSOL 1789 et<br>les produits vendus sous les marques EUSOLEX 232, UNIVUL T 150, UNIVUL N 539, ESCALOL 507. |

TABLEAU I (suite 4)

| FONCTION | ACTIFS UTILISABLES |
|---|---|
| Kératolytique | L'acide salicylique et ses dérivés, tels que les acides alkylsalicyliques, notamment l'acide n-octanoyl-5 salicylique et le n-dodécanoyl-5, salicylate de N-hexadécyl pyridinium.<br>L'acide rétinoïque.<br>Les enzymes protéolytiques, notamment la trypsine, l'alpha-chymotrypsine, la papaïne, la bromelaine et la pepsine.<br>Le peroxyde de benzoyle.<br>L'urée.<br>Les alpha-hydroxyacides. |
| Emollient | Esters tels que l'adipate d'isopropyle. |
| Anti-inflammatoire | Les corticoïdes tels que le 17-acétate de ß-méthasone, l'indométhacine, le ketoprofène, l'acide flufenamique, l'ibuprofene, le dichlofenac, le diflunisal, le fenclofenac, le naproxene, le piroxidam, et le sulindac.<br>Le monostéaryl éther de glycérol (alcool batylique) et le mono-cétyléther de glycérol (alcool chimylique).<br>L'acide glycyrrhétinique et ses sels, notamment d'ammonium.<br>L'alpha-bisabolol (extrait de camomille).<br>La shikonine.<br>Des extraits de plantes, tels qu'eau de bleuet, arnica, aloès.<br>Des extraits de tissu méristématique, notamment l'extrait de racine de chêne.<br>Le plancton. |
| Rafraichissant | Le menthol.<br>Le lactate de menthyle. |
| Cicatrisant | Extrait de mimosa tenuiflora.<br>L'extrait de Centella Asiatica.<br>L'acide ß-glycyrrhétinique.<br>L'hydroxyproline.<br>L'arginine.<br>Un extrait placentaire.<br>Un extrait de levure.<br>Le fagaramide.<br>La N-acétyl hydroxyproline.<br>L'acide acexamique et ses dérivés. |

## TABLEAU I (suite 5)

| FONCTION | ACTIFS UTILISABLES |
|---|---|
| Protecteur vasculaire | Les flavonoïdes, notamment les dérivés de rutine, tels que l'étoxazorutine et le rutine propylsulfonate de sodium.<br>Les extraits végétaux, notamment l'extrait huileux de Ginkgo biloba, l'extrait de marron d'Inde (escine), de lierre (saponines) et de petit houx.<br>Le nicotinate d'alpha-tocophérol. |
| Anti-bactérien, antifongique | Le bromure de triméthylcétylammonium.<br>L'acide sorbique.<br>Le peroxyde de benzoyle.<br>Le chlorure de cétylpyridinium.<br>Le chlorure de benzalkonium.<br>L'acide parahydroxybenzoïque et ses sels.<br>Le bromo-2 nitro-2 propanediol-1,3.<br>Le trichloro-3,4,4'-carbanilide.<br>Le trichloro-2,4,4'-hydroxy-2 diphényléther.<br>L'acide déhydroacétique.<br>Un extrait de pamplemousse dans la glycérine et le propylène glycol.<br>La chlorhexidine.<br>L'hexetidine.<br>L'hexamidine. |
| Agent insectifuge | Le diméthyltoluamide. |
| Antiperspirant | Le chlorhydrate d'aluminium.<br>Le chlorure d'aluminium.<br>Le complexe lactate de sodium/aluminium chlorhydroxy.<br>Le chlorhydrate de zirconyle. |

## TABLEAU I (suite 6)

| FONCTION | ACTIFS UTILISABLES |
|---|---|
| Déodorant | L'oxyde de zinc.<br>Le ricinoléate de zinc.<br>L'éthyl-2 hexanediol-1,3.<br>L'hexachlorophène.<br>Le produit vendu sous la marque "IRGASAN DP 300". |
| Anti-pelliculaire | L'octopyrox.<br>Les omadines.<br>Le goudron de houille.<br>L'hydroxy-1 méthyl-4 triméthyle-2,4,4 pentyl-6 pyridinone-2.<br>Le sulfure de sélénium. |
| Anti-chute des cheveux | Les inhibiteurs de glucuronidases.<br>Les muccopolysaccharides.<br>Le nicotinate de méthyle ou d'hexyle.<br>La forskoline.<br>Le minoxidil.<br>Les xanthines.<br>Les rétinoïdes. |
| Colorant capillaire | Les bases et coupleurs d'oxydation.<br>Les colorants directs.<br>Les colorants auto-oxydables. |
| Agent décolorant pour cheveux | Le peroxyde d'hydrogène. |
| Réducteur pour permanentes | L'acide thioglycolique.<br>La cystéine.<br>La cystéamine.<br>La N-acétyl cystéine.<br>La N-acétyl cystéamine.<br>Le thioglycolate de glycérol. |
| Agent conditionneur pour peau et cheveux | Polymères cationiques, cations. |

Les différents actifs introduits peuvent être tous liposolubles, hydrosolubles ou amphiphiles ou appartenir à au moins deux de ces catégories. Les actifs introduits peuvent avoir la même fonction ou des fonctions différentes. Il faut noter que certains actifs ont plusieurs fonctions.

La phase aqueuse de la dispersion de la composition selon l'invention peut également contenir une dispersion de gouttelettes d'un liquide non miscible à l'eau, comme décrit dans les brevets français 2 485 921 et 2 490 504. On a, en effet, constaté que les vésicules selon l'invention stabilisent la dispersion de gouttelettes de liquide non miscible à l'eau sans qu'il soit nécessaire d'introduire un émulsionnant conventionnel.

Le liquide non miscible à l'eau, qui peut être présent sous forme de dispersion dans la phase aqueuse de dispersion, peut notamment être choisi dans le groupe formé par :

- les huiles animales ou végétales formées par des esters d'acide gras et de polyols, en particulier les triglycérides liquides, par exemple les huiles de tournesol, de maïs, de soja, de courge, de pépins de raisin, de jojoba, de sésame, de noisette, les huiles de poisson, le tri-caprocaprylate de glycérol, ou les huiles végétales ou animales de formule $R_9COOR_{10}$, formule dans laquelle $R_9$ représente le reste d'un acide gras supérieur comportant de 7 à 19 atomes de carbone et $R_{10}$ représente une chaîne hydrocarbonée ramifiée contenant de 3 à 20 atomes de carbone, par exemple l'huile de Purcellin;
- des huiles essentielles naturelles ou synthétiques telles que, par exemple, les huiles d'eucalyptus, de lavandin, de lavande, de vétiver, de litsea cubeba, de citron, de santal, de romarin, de camomille, de sarriette, de noix de muscade, de cannelle, d'hysope, de carvi, d'orange, de géraniol, de cade et de bergamote;
- des hydrocarbures, tels que l'hexadécane et l'huile de paraffine;
- des carbures halogénés, notamment des fluorocarbures tels que des fluoroamines par exemple la perfluorotributylamine, des hydrocarbures fluorés, par exemple le perfluorodécahydronaphtalène, des fluoroesters et des fluoroéthers ;
- des silicones, par exemple, les polysiloxanes, les polydiméthylsiloxanes et les fluorosilicones ;
- des esters d'acide minéral et d'un alcool ; et
- des éthers et des polyéthers.

La phase aqueuse de dispersion peut aussi également contenir des actifs cosmétiques et/ou dermopharmaceutiques, hydrosolubles. Le liquide non-miscible à l'eau peut éventuellement contenir un actif liposoluble.

La phase aqueuse de dispersion peut aussi contenir des adjuvants n'ayant ni activité cosmétique ni activité dermopharmaceutique propre, mais utilisés pour la formulation de la dispersion sous forme de lotion, crème ou sérum. Ces adjuvants sont, en particulier, pris dans le groupe formé par les gélifiants, les conservateurs, les colorants, les pigments, les charges, les opacifiants et les parfums.

Parmi les gélifiants utilisables, on peut citer les dérivés de cellulose tels que l'hydroxyéthylcellulose, les dérivés d'algues tels que le satiagum, des gommes naturelles, telles que l'adragante, et des polymères synthétiques, en particulier les mélanges d'acides polycarboxyvinyliques commercialisés sous la dénomination "CARBOPOL" par la société "GOODRICH"et le mélange de copolymères acrylate de Na/acrylamide commercialisé sous la dénomination "HOSTACERIN PN 73" par la société "HOECHST".

Parmi les pigments utilisables selon l'invention, on peut, en particulier, citer les pigments enrobés par des silicones, par des composés fluorés, tels que les perfluoroalkylphosphates ou le polytétrafluoroéthylène (vendu sous la marque commerciale "TEFLON"), ou par des amino-acides ; ces pigments enrobés peuvent être, notamment, des oxydes de fer ou de titane. A titre d'exemple, on peut citer les produits vendus par la société "WACKHERR" sous la dénomination commerciale "COVAFLUOR", qui sont notamment des oxydes de fer ou de titane enrobés par du perfluoroalkylphosphate ; on peut également citer les produits vendus par la société "CLARK COLOR" sous la dénomination commerciale "TEFLON TREATED PIGMENTS", qui sont notamment des oxydes de fer ou de titane enrobés par du polytétrafluoroéthylène.

Parmi les charges utilisables selon l'invention, on peut, en particulier, citer le talc, le mica, la poudre d'amidon, la poudre de nylon et la poudre de silice.

Dans la composition selon l'invention, les vésicules ont généralement un diamètre moyen compris entre 10 et 5 000 nm. Lorsque la phase aqueuse contient une dispersion de gouttelettes de liquide non miscible à l'eau, ces gouttelettes ont de façon avantageuse un diamètre moyen compris entre 100 et 10 000 nm.

Les exemples ci-après, donnés à titre purement illustratif et non limitatif, permettront de mieux comprendre l'invention.

Dans tous les exemples donnés ci-après, les dispersions de vésicules sont préparées par le procédé dit "par cofusion des lipides" dans lequel :

- dans une première phase, on prépare la phase lipidique par mélange sous forme liquide des différents lipides amphiphiles la composant, et éventuellement associés à des additifs ou des actifs liposolubles, et on met la phase lipidique obtenue en présence d'une phase aqueuse contenant éventuellement des actifs hydrosolubles, de façon à obtenir une phase lamellaire,
- dans une deuxième phase, on ajoute à la phase lamellaire hydratée obtenue une phase aqueuse de dispersion contenant éventuellement un liquide non miscible à l'eau et différents additifs,

- et dans une troisième phase, on soumet le mélange à une agitation énergique dans un homogénéiseur pour obtenir des vésicules dispersées dans une phase aqueuse de dispersion.

**EXEMPLE 1**

On a préparé, à titre comparatif, des vésicules à partir de phase lipidique contenant, comme lipide non-ionique, l'un ou l'autre des deux produits A et B suivants :

A) Palmitate de sorbitane, commercialisé par la société "ICI" sous la dénomination "SPAN 40", qui est un mélange contenant en majeure partie du monopalmitate de sorbitane et de faibles quantités de di-, tri- et tétrapalmitate de sorbitane ;
B) Le composé chimiquement pur, tel qu'utilisé dans l'exemple 4 de EP-A-0 433 131, de formule :

$$C_{15}H_{31}CO(OCH_2CHOHCH_2)_2OH$$

Lorsqu'on utilise le produit A, la composition obtenue comme indiqué ci-après est dans le cadre de l'invention alors qu'elle ne l'est pas avec le produit B.

La composition a la formulation suivante :

| | |
|---|---|
| - Lipide non-ionique A ou B | 1,425 g |
| - Cholestérol | 1,425 g |
| - Glutamate monosodique commercialisé sous la dénomination "ACYLGLUTAMATE HS 11" par la société "AJINOMOTO" | 0,15 g |
| - Conservateur | 0,02 g |
| - Eau qsp | 100 g |

On a obtenu après homogénéisation une dispersion contenant des vésicules de 250 nm.

On a soumis les deux dispersions obtenues à un test de stabilité en les soumettant à 5 cycles successifs de variation de température entre - 20°C et + 20°C, chaque cycle se déroulant de la façon suivante :

- 10 heures à -20°C
- 2 heures de -20° à + 20°C
- 10 heures à + 20°C.

Les changements de température s'effectuent avec un gradient de 0,33 °C/mn

A l'issue des cycles, on a observé, à l'oeil et au microscope optique, la dispersion de vésicules contenant le lipide nonionique A selon l'invention et celle de vésicules contenant le lipide non-ionique B chimiquement pur (composition ne faisant pas partie de l'invention). On a constaté que les vésicules contenant le lipide non-ionique A selon l'invention sont intactes, tandis que les vésicules contenant le lipide non-ionique B sont massivement recristallisées.

**EXEMPLES 2 à 6**

On a préparé, à titre comparatif, des compositions contenant une dispersion de vésicules dont la phase lipidique contient un lipide ionique faisant partie de l'invention et, des compositions contenant une dispersion dont la phase lipidique contient un lipide ionique ne faisant pas partie de l'invention.

La formulation des phases lipidiques utilisées dans les différents exemples est donnée dans le tableau II ci-après :

TABLEAU II

| | Exemples | | | | |
|---|---|---|---|---|---|
| | 2 | 3 | 4 | 5 | 6 |
| Stéarate de tétraglycérol commercialisé par la société "NIKKO" | 47,5 | - | 47,5 | - | - |
| Isostéarate de diglycéryle commercialisé par la société "SOLVAY" | - | 90 | - | 90 | - |
| Palmitate de sorbitane commercialisé sous la dénomination "SPAN 40" par la société "ICI" | - | - | - | - | 45 |
| Cholestérol | 47,5 | - | 47,5 | - | 45 |
| Dicétylphosphate acide | 5 | 10 | | | |
| Glutamate commercialisé sous la dénomination "ACYLGLUTAMATE HS 11" par la société "AJINOMOTO" | | | 5 | 10 | 10 |
| pH de la dispersion aqueuse(1) (1)Le pH a été mesuré sur des dispersions contenant 2% de lipide et 0,1% de conservateur. | 3,3 | 3,2 | 6,3 | 6,3 | 6,1 |

On a mesuré à l'aide d'un granulomètre laser AMTECH BI 90 le diamètre des vésicules au temps T0, juste après leur formation, après 2 mois de stockage à température ambiante (TA), à 37°C et à 45°C ; les résultats sont donnés dans le tableau III ci-après :

**TABLEAU III**

**DIAMETRE MOYEN DES VESICULES EN MICRON**

| Associations lipidiques testées | au temps T0 | après 2 mois | | |
|---|---|---|---|---|
| | | à TA | à 37°C | à 45°C |
| Exemple 2 | 0,24 | S | C | C++ |
| Exemple 3 | 0,09 | S | S | 0.18 - I |
| Exemple 4 | 0,25 | S | S | S |
| Exemple 5 | 0,19 | S | S | S |
| Exemple 6 | 0,24 | S | S | S |

S : vésicules de diamètre stable
I : vésicules de diamètre instable
C : présence de cristaux
C++ : présence massive de cristaux

Ce tableau III montre que la présence d'un lipide anionique acide ne faisant pas partie de l'invention (exemples 2 et 3) ne permet pas de stabiliser les vésicules, tandis qu'un lipide anionique neutralisé selon l'invention (exemples 4 à 6) permet de stabiliser les vésicules.

**EXEMPLE 7** : Biodégradabilité des vésicules selon l'invention sous l'effet des enzymes des couches superficielles de la peau humaine.

On a préparé une composition vésiculaire ayant la formulation suivante (en % en poids) :

| | |
|---|---|
| - Palmitate de sorbitane vendu sous la dénomination "SPAN 40" par la société "ICI" | 1,425 % |
| - Cholestérol | 1,425 % |
| - Glutamate monosodique commercialisé sous la dénomination "ACYLGLUTAMATE HS 11" par la société "AJINOMOTO" | 0,15 % |
| - Glycérine | 3,00 % |
| - Conservateur $NaN_3$ | 0,02 % |
| - Eau qsp | 100 % |

Le pH de la phase aqueuse de dispersion est de 6,0 et le diamètre des vésicules est de 260 nm.

On a préparé, par ailleurs, un jus enzymatique par traitement d'une surface de la peau du dos de 4 x 4 cm ayant subi 8 strips successifs. Le matériel enzymatique est extrait par l'acétone puis lyophilisé. La poudre obtenue est dissoute dans 0,5 ml de la dispersion de vésicules ci-dessus.

On dose l'acide palmitique libéré dans le mélange réactionnel après 0 à 7 jours d'incubation à 37°C. Le dosage de l'acide palmitique libéré est effectué par deux procédés :

1) par chromatographie en couche mince haute performance dans les conditions suivantes :

. Eluant : Hexane 80 - Ether de pétrole 20 - Acide acétique 3 (en volume)
. Révélateur : Sulfate de cuivre à 4 % (en poids) dans l'acide orthophosphorique à 15 % (en poids)
. Traitement : Carbonisation à 180°C pendant 5 minutes
. Lecture : Densitomètre SHIMADZU à 540 nm.

2) à l'aide du kit enzymatique BOHRINGER n°10 82 914 de dosage des acides gras libres étalonnés sur l'acide palmitique.

Les deux dosages donnent des résultats concordants.

Dans le tableau IV ci-après, la quantité d'acide palmitique libérée est donnée en % par rapport au total présent au départ sous forme de palmitate de sorbitane.

TABLEAU IV

| Temps d'incubation | Acide palmitique |
|---|---|
| 15 heures | 3 % |
| 3 jours | 23 % |
| 7 jours | 40 % |

Les vésicules selon l'invention permettent donc une libération retardée des actifs présents dans les vésicules, soit dans la phase aqueuse encapsulée, soit dans la membrane lipidique.

**EXEMPLE 8**: Crème de jour pour le visage destinée au soin des peaux sèches.

La crème est préparée par le procédé défini ci-dessus.

*Dans une première phase*, la formulation utilisée pour la composition vésiculaire est la suivante :

| | |
|---|---|
| - Palmitate de sorbitane commercialisé sous la dénomination de "SPAN 40" par la société "ICI" | 3,8 g |
| - Cholestérol | 3,8 g |
| - Sel monosodique de l'acide N-stéaroyl glutamique commercialisé sous la dénomination "ACYLGLUTAMATE HS 11" par la société "AJINOMOTO" | 0,4 g |
| - Eau déminéralisée qsp | 50 g |
| - Conservateurs | 0,3 g |
| - Glycérine | 5,0 g |

*Dans une deuxième phase*, la formulation de la dispersion d'huile est la suivante :

| | |
|---|---|
| - Huile de macadamia | 16,0 g |
| - Parfum | 0,2 g |
| - Mélange d'acides polycarboxyvinyliques commercialisé sous la dénomination de "CARBOPOL 940" par la société "GOODRICH" | 0,42g |
| - Triéthanolamine qs | pH 6 |
| - Eau déminéralisée qsp | 100 g |

*Dans une troisième phase*, on mélange comme indiqué précédemment les deux formulations pour obtenir la composition selon l'invention.

Après une semaine d'application quotidienne, chaque matin, sur le visage soigneusement nettoyé, on constate une amélioration de l'aspect et de l'éclat de la peau du visage qui devient plus lisse, plus ferme, plus hydratée, avec un teint plus éclatant.

**EXEMPLE 9**: Crème de jour anti-âge pour le visage.

On prépare, comme dans l'exemple 8, une crème ayant la formulation suivante :

*Première phase :*

| | |
|---|---|
| - Stéarate de sorbitane commercialisé sous la dénomination de "SPAN 60" par la société "ICI" | 3,8 g |
| - Cholestérol | 3,8 g |
| - Sel monosodique de l'acide N-stéaroyl glutamique commercialisé sous la dénomination "ACYLGLUTAMATE HS 11" par la société "AJINOMOTO" | 0,4 g |
| - Glycérine | 3,0 g |
| - L-Hydroxyproline | 1,0 g |
| - D-Panthenol | 1,5 g |
| - Guanosine | 0,01 g |
| - Eau déminéralisée qsp | 50 g |
| - Conservateurs | 0,3 g |
| - Polyphosphonate commercialisé sous la dénomination de "DEQUEST 2046" par la société "MONSANTO CHEMICAL" | 0,8 g |
| - Hydrolysat lactique commercialisé sous la dénomination de "LACTOLAN LS" par les "Laboratoires Sérobiologiques de Nancy" | 5,0 g |
| - L-Sérine | 0,2 g |

*Deuxième phase* :

| | |
|---|---|
| - Paraoxybenzoate de propyle | 0,3 g |
| - Huile de macadamia | 7,0 g |
| - Concentrats naturels de tocophérols | 4,0 g |
| - Filtres antisolaires | 1,0 g |
| - Huile de silicone volatile | 7,5 g |
| - Glycérides de vitamine F | 3,0 g |
| - Mélange d'acides polycarboxyvinyliques commercialisé sous la dénomination de "CARBOPOL 940" par la société "GOODRICH" | 0,5 g |
| - Paraoxybenzoate de méthyle | 0,2 g |
| - Triéthanolamine qs | pH 6,5 |
| - Eau déminéralisée qsp | 100 g |

Après une semaine d'application quotidienne, chaque matin, sur le visage soigneusement nettoyé, on constate une amélioration de l'aspect et de l'éclat de la peau du visage, qui devient plus lisse, plus ferme, plus hydratée, avec un teint plus éclatant.

**EXEMPLE 10** : Sérum anti-âge pour le visage.

On prépare un sérum ayant la formulation suivante :

| | |
|---|---|
| - Laurate de sorbitane commercialisé sous le nom de "SPAN 20" par la société "ICI" | 1,5 g |
| - Cholestérol | 0,4 g |
| - Sel monosodique de l'acide N-stéaroyl glutamique commercialisé sous le nom "ACYLGLUTAMATE HS 11" par la société "AJINOMOTO" | 0,1 g |
| - Glycérine | 3,0 g |
| - L-Hydroxyproline | 1,0 g |
| - D-Panthenol | 1,5 g |
| - Guanosine | 0,01 g |
| - Conservateurs | 0,3 g |
| - Polyphosphonate commercialisé sous la dénomination de "DEQUEST 2046" par la société "MONSANTO CHEMICAL" | 0,8 g |
| - Hydrolysat lactique commercialisé sous la dénomination de "LACTOLAN LS" par les "Laboratoires Sérobiologiques de Nancy" | 5,0 g |
| - L-Sérine | 0,2 g |
| - Solution aqueuse de superoxyde dismutase à 5 000 unités par ml commercialisée par la société "PENTAPHARM" | 1,0 g |
| - Mélange d'acides polycarboxyvinyliques commercialisé sous la dénomination "CARBOPOL 940" par la société "GOODRICH" | 0,3 g |
| - L-Lysine monohydrate qs | pH 6,5 |
| - Eau déminéralisée qsp | 100 g |

Après application deux fois par jour, pendant trois semaines, on note un net raffermissement de la peau.

**EXEMPLE 11** : Sérum anti-âge pour le visage.

On prépare un sérum ayant la formulation suivante :

| | |
|---|---|
| - Oléate de sorbitane commercialisé sous la dénomination de "SPAN 80" par la société "ICI" | 0,75 g |
| - Cholestérol | 0,20 g |
| - Sel de sodium de l'acide phosphatidique | 0,05 g |
| - Glycérine | 3,0 g |
| - L-Hydroxyproline | 1,0 g |
| - D-Panthenol | 1,5 g |
| - Guanosine | 0,01 g |
| - Conservateurs | 0,3 g |
| - Polyphosphonate commercialisé sous la dénomination de "DEQUEST 2046" par la société "MONSANTO CHEMICAL" | 0,8 g |
| - Hydrolysat lactique commercialisé sous la dénomination de "LACTOLAN LS" par les "Laboratoires Sérobiologiques de Nancy" | 5,0 g |
| - L-Sérine | 0,2 g |
| - Solution aqueuse de superoxyde dismutase à 5 000 unités par ml commercialisée par la société "PENTAPHARM" | 1,0 g |
| - Mélange d'acides polycarboxyvinyliques commercialisé sous la dénomination de "CARBOPOL 940" par la société "GOODRICH" | 0,3 g |
| - L-Lysine monohydrate qs | pH 6,5 |
| - Eau déminéralisée qsp | 100 g |

On a observé que les vésicules contenues dans le sérum sont restées stables après 3 mois de stockage. Après application 2 fois par jour, pendant 3 semaines, on note un net raffermissement de la peau.

**EXEMPLE 12** : Crème de jour pour le visage.

On a préparé une crème ayant la formulation suivante :

*Première phase :*

| | |
|---|---|
| - Stéarate de sorbitane oxyéthyléné 4 moles commercialisé sous la dénomination de "TWEEN 61" par la société "ICI" | 3,8 g |
| - Cholestérol | 3,8 g |
| - Sel monosodique de l'acide N-stéaroyl glutamique commercialisé sous la dénomination "ACYLGLUTAMATE HS 11" par la société "AJINOMOTO" | 0,4 g |
| - Glycérine | 5,0 g |
| - Conservateurs | 0,3 g |
| - Eau déminéralisée qsp | 50 g |

*Deuxième phase* :

| | |
|---|---|
| - Huile de macadamia | 16,0 g |
| - Parfum | 0,2 g |
| - Mélange d'acides polycarboxyvinyliques commercialisé sous la dénomination de "CARBOPOL 940" par la société "GOODRICH" | 0,42 g |
| - Triéthanolamine qs | pH 6 |
| - Eau déminéralisée qsp | 100 g |

Après une semaine d'application quotidienne, chaque matin, sur le visage soigneusement nettoyé, on constate une amélioration de l'aspect et de l'éclat de la peau du visage, qui devient plus lisse, plus ferme, plus hydratée, avec un teint plus éclatant.

**EXEMPLE 13** : Crème de jour pour le visage.

On a préparé une crème ayant la formulation suivante :

*Première phase* :

| | |
|---|---|
| - Stéarate de sorbitane oxyéthyléné 8 moles commercialisé sous la dénomination de "MYRJ 45" par la société "ICI" | 4,4 g |
| - Cholestérol | 3,2 g |
| - Sel disodique de l'acide N-stéaroyl glutamique commercialisé sous la dénomination "ACYLGLUTAMATE HS 21" par la société "AJINOMOTO" | 0,4 g |
| - Glycérine | 5,0 g |
| - Conservateurs | 0,3 g |
| - Eau déminéralisée qsp | 50 g |

*Deuxième phase* :

| | |
|---|---|
| - Huile de macadamia | 16,0 g |
| - Parfum | 0,2 g |
| - Mélange d'acides polycarboxyvinyliques commercialisé sous la dénomination de "CARBOPOL 940" par la société "GOODRICH" | 0.42 g |
| - Triéthanolamine qs | pH 6 |
| - Eau déminéralisée qsp | 100 g |

Après une semaine d'application quotidienne, chaque matin, sur le visage soigneusement nettoyé, on constate une amélioration de l'aspect et de l'éclat de la peau du visage, qui devient plus lisse, plus ferme, plus hydratée, avec un teint plus éclatant.

**EXEMPLE 14** : Crème de jour pour le visage.

On prépare une crème ayant la formulation suivante :

*Première phase* :

| | |
|---|---|
| - Stéarate de tétraglycérol commercialisé sous la dénomination de "TETRAGLYN 1 S" par la société "NIKKO" | 3,8 g |
| - Cholestérol | 3,8 g |
| - Sel monosodique de l'acide N-stéaroyl glutamique commercialisé sous la dénomination "ACYLGLUTAMATE HS 11" par la société "AJINOMOTO" | 0,4 g |
| - Glycérine | 5,0 g |
| - Conservateurs | 0,3 g |
| - Eau déminéralisée qsp | 50 g |

*Deuxième phase* :

| | |
|---|---|
| - Huile de macadamia | 16,0 g |
| - Parfum | 0,2 g |
| - Mélange d'acides polycarboxyvinyliques commercialisé sous la dénomination de "CARBOPOL 940" par la société "GOODRICH" | 0,42 g |
| - Triéthanolamine qs | pH 6 |
| - Eau déminéralisée qsp | 100 g |

Après une semaine d'application quotidienne, chaque matin, sur le visage soigneusement nettoyé, on constate une amélioration de l'aspect et de l'éclat de la peau du visage, qui devient plus lisse, plus ferme, plus hydratée, avec un teint plus éclatant.

**EXEMPLE 15**: Crème de jour pour le visage.

On prépare une crème ayant la formulation suivante :

*Première phase* :

- Palmitate de sorbitane commercialisé sous la dénomination de "SPAN 40" par la société "ICI" .......... 1,0 g

- Céramide de synthèse répondant à la formule

$$R_1CHOHCHCH_2OH \quad (I)$$

$$NHCOR_2$$

pour laquelle $R_1$ et $R_2$ sont $C_{15}H_{31}$ et qui est un mélange des formes érythro et thréo dans la proportion 70 : 30 .............................. 1,0 g
- Cholestérol ....................................................... 0,7 g
- Sel de sodium du cholestérol sulfate ......................... 0,30 g
- Glycérine ........................................................ 3,0 g
- Conservateurs .................................................... 0,3 g
- Laurate de sorbitane oxyéthyléné à 20 moles d'O.E. commercialisé sous le nom de "TWEEN 20" par la société "ICI" ............................ 1,0 g
- Eau déminéralisée ....................... qsp .................. 50 g

*Deuxième phase*

| | |
|---|---|
| - Huile de silicone volatile | 10,0 g |
| - Huile d'amandes d'abricots | 10,0 g |
| - Parfum | 0,2 g |
| - Mélange d'acides polycarboxyvinyliques commercialisé sous la dénomination de "CARBOPOL 940" par la société "GOODRICH" | 0,42g |
| - Triéthanolamine qs | pH 6,5 |
| - Eau déminéralisée qsp | 100 g |

Cette crème permet, après décomposition du palmitate de sorbitane et du laurate de sorbitane, par les enzymes de la peau, de traiter la peau par l'acide palmitique, l'acide laurique, le sorbitol, un céramide et du cholestérol, et fournit à la peau une protection particulièrement efficace. Cette crème appliquée quotidiennement, pendant 20 jours, sur une peau fatiguée, donne des résultats appréciables.

**EXEMPLE 16** : Sérum anti-âge.

On prépare un sérum ayant la formulation suivante :

- Palmitate de sorbitane commercialisé sous la dénomination de "SPAN 40" par la société "ICI" .......... 0,17 g
- Céramide de synthèse répondant à la formule

$$\begin{array}{l} R_1CHOHCHCH_2OH \\ \qquad\quad\;| \\ \qquad\quad NHCOR_2 \end{array} \qquad (I)$$

pour laquelle $R_1$ est $C_{15}H_{31}$ et R2 est le reste du chlorure d'oléyle commercialisé par la société "BASF" avec un titre en chlorure d'acide de 98% et qui est un mélange des formes érythro et thréo dans la proportion 70 : 30 .............................................. 0,17 g
- Cholestérol ...................................................... 0,11 g
- Cholestérol phosphate (sel de sodium) ....................... 0,05 g
- Glycérine ......................................................... 5,0 g
- Laurate de sorbitane oxyéthyléné à 20 moles d'O.E. commercialisé sous le nom de "TWEEN 20" par la société "ICI" ............................ 0,17 g
- Conservateurs .................................................. 0,3 g
- Palmitate d'ascorbyle ......................................... 0,01 g
- Polyéthylène glycol (poids moléculaire = 400) ........... 1,0 g
- Propylène glycol ............................................... 3,0 g
- Eau ................................................................ 50,0 g
- Hyaluronate de sodium ........................................ 0,1 g
- Eau ................................................................ 15,0 g
- Mélange d'acides polycarboxyvinyliques commercialisé sous la dénomination de "CARBOPOL 940" par la société "GOODRICH" ............ 0,25 g
- Triéthanolamine ......................... qs ................ pH 6,5
- Eau déminéralisée ....................... qsp ............... 100 g

Ce sérum permet, après décomposition par les enzymes de la peau, du palmitate de sorbitane et du laurate de sorbitane, de traiter la peau par l'acide palmitique, l'acide laurique, le sorbitol, un céramide et du cholestérol, et fournit à la peau une protection particulièrement efficace. Ce sérum appliqué quotidiennement, pendant 20 jours, sur une peau fatiguée, donne des résultats appréciables.

<u>EXEMPLE 17</u> : Crème de jour pour le visage.

On prépare une crème ayant la formulation suivante :

*Première phase*

| | |
|---|---|
| - Palmitate de sorbitane commercialisé sous la dénomination de "SPAN 40" par la société "ICI" | 1,4 g |
| - Glycocéramides de germes de blé commercialisés par la société "ARD" | 1,4 g |
| - Cholestérol | 0,90 g |
| - Cholestérol sulfate (sel de sodium) | 0,30 g |
| - Glycérine | 4,0 g |
| - Eau déminéralisée qsp | 56 g |

*Deuxième phase*

| | |
|---|---|
| - Huile de silicone volatile | 10,0 g |
| - Huile d'amandes d'abricots | 10,0 g |
| - Mélange d'acides polycarboxyvinyliques commercialisé sous la dénomination de "CARBOPOL 940" par la société "GOODRICH" | 0,42 g |
| - Paraoxybenzoate de méthyle | 0,20 g |
| - Triéthanolamine qs | pH 6,5 |
| - Eau déminéralisée qsp | 100 g |

Après une semaine d'application quotidienne, chaque matin, sur le visage soigneusement nettoyé, on constate une amélioration de l'aspect et de l'éclat de la peau du visage, qui devient plus lisse, plus ferme, plus hydratée, avec un teint plus éclatant.

**EXEMPLE 18** : Lait parfumé pour le corps.

On prépare un lait ayant la formulation suivante :

*Première phase* :

| | |
|---|---|
| - Isostéarate de diglycéryle commercialisé par la société "SOLVAY" | 0,9 g |
| - Sel monosodique de l'acide N-stéaroyl glutamique commercialisé sous la dénomination "ACYLGLUTAMATE HS 11" par la société "AJINOMOTO" | 0,1 g |
| - Glycérine | 3,0 g |
| - Conservateurs | 0,3 g |
| - Eau déminéralisée qsp | 69,7 g |

*Deuxième phase* :

| | |
|---|---|
| - Huile essentielle de bergamote (exempte de bergaptène) | 5,0 g |
| - Mélange d'acides polycarboxyvinyliques commercialisé sous la dénomination de "CARBOPOL 940" par la société "GOODRICH" | 0,10 g |
| - Triéthanolamine qs | pH 6 |
| - Eau déminéralisée qsp | 100 g |

Ce lait appliqué une fois par jour sur le corps sur des sujets à peau ferme donne une amélioration de l'aspect et de l'éclat de la peau, qui devient plus lisse et plus hydratée, de façon appréciable, après 20 jours d'application.

**EXEMPLE 19**: Crème de jour pour le visage destinée à la nutrition des peaux ternes.

On prépare une crème ayant la formulation suivante :

*Première phase* :

| | |
|---|---|
| - Stéarate de polyéthylène glycol "PEG 400" produit par biotechnologie commercialisé par la société "UNICHEMA" | 1,9 g |
| - Cholestérol | 1,9 g |
| - Sel monosodique de l'acide N-stéaroyl glutamique commercialisé sous la dénomination "ACYLGLUTAMATE HS 11" par la société "AJINOMOTO" | 0,2 g |
| - Sorbitol | 4,0 g |
| - Mélange d'acides aminés, manitol, saccharose végétal, glycogène marin, acide pyrrolidone carboxylique non stabilisé commercialisé sous la dénomination "HYDROSMYL LS 4513" par les "Laboratoires Sérobiologiques de Nancy" | 0,5 g |
| - Conservateurs | 0,3 g |
| - Eau déminéralisée | |

*Deuxième phase* :

| | |
|---|---|
| - Huile de pépins de raisin | 8,0 g |
| - Huile de tournesol | 8,0 g |
| - Huile de silicone volatile | 4,0 g |
| - Parfum | 0,2 g |
| - Mélange d'acides polycarboxyvinyliques commercialisé sous la dénomination de "CARBOPOL 940" par la société "GOODRICH" | 0,42 g |
| - Triéthanolamine qs | pH 6 |
| - Eau déminéralisée qsp | 100 g |

Après une semaine d'application quotidienne, chaque matin, sur le visage soigneusement nettoyé, on constate une amélioration de l'éclat de la peau du visage, qui devient plus éclatant.

**EXEMPLE 20**: Lait hydratant pour le corps

On prépare un lait ayant la formulation suivante :

*Première phase* :

| | |
|---|---|
| - Distéarate de sucrose commercialisé sous la dénomination "CRODESTA F 50" par la société "CRODA" | 1,35 g |
| - Cholestérol | 1,35 g |
| - Sel disodique de l'acide N-stéaroyl glutamique commercialisé sous la dénomination "ACYLGLUTAMATE HS 21" par la société "AJINOMOTO" | 0,30g |
| - Glycérine | 3,0 g |
| - Conservateurs | 0,30 g |
| - Eau déminéralisée | |

*Deuxième phase* :

| | |
|---|---|
| - Huile de vaseline légère | 6,0 g |
| - Huile d'amande douce | 4,0 g |
| - Parfum | 0,20 g |
| - Mélange d'acides polycarboxyvinyliques commercialisé sous la dénomination de "CARBOPOL 940" par la société "GOODRICH" | 0,40 g |
| - Triéthanolamine qs | pH 6,5 |
| - Eau déminéralisée qsp | 100 g |

Ce lait appliqué une fois par jour sur le corps sur des sujets à peau ferme donne une amélioration de l'aspect et de l'éclat de la peau, qui devient plus lisse et plus hydratée, de façon appréciable, après 20 jours d'application.

**EXEMPLE 21**: Crème de jour anti-âge pour le visage.

On prépare une crème ayant la formulation suivante :

*Première phase* :

| | |
|---|---|
| - Distéarate de sucrose commercialisé sous la dénomination "GRILLOTEN PSE 141 G" par la société "GRILLO" | 2,70 g |
| - Cholestérol | 2,70 g |
| - Sel disodique de l'acide N-stéaroyl glutamique commercialisé sous la dénomination "ACYLGLUTAMATE HS 21" par la société "AJINOMOTO" | 0,60 g |
| - Acétate de vitamine E | 0,60 g |
| - Glycérine | 3,00 g |
| - L-Hydroxyproline | 1,00 g |
| - Guanosine | 0,02 g |
| - Conservateurs | 0,30 g |
| - Polyphosphonate commercialisé sous la dénomination de "DEQUEST 2046" par la société "MONSANTO CHEMICAL" | 0,80 g |
| - Hydrolysat lactique commercialisé sous la dénomination de "LACTOLAN LS" par la société "Laboratoires Sérobiologiques de Nancy" | 5,00 g |
| - Eau déminéralisée | 40 g |

*Deuxième phase* :

| | |
|---|---|
| - Huile d'amandes d'abricots | 10,0 g |
| - Huile de silicone volatile | 10,0 g |
| - Filtres antisolaires | 1,00 g |
| - Glycérides de vitamine F | 2,00 g |
| - Mélange d'acides polycarboxyvinyliques commercialisé sous la dénomination de "CARBOPOL 940" par la société "GOODRICH" | 0,50 g |
| - Conservateurs | 0,20 g |
| - Triéthanolamine qs | pH 6,5 |
| - Eau déminéralisée qsp | 100 g |

Après une semaine d'application quotidienne, chaque matin, sur le visage soigneusement nettoyé, on constate une amélioration de l'aspect et de l'éclat de la peau du visage, qui devient plus lisse, plus ferme, plus hydratée, avec un teint plus éclatant.

**EXEMPLE 22**: Crème de jour pour le visage et le cou destinée au soin des peaux fatiguées.

On prépare une crème ayant la formulation suivante :

*Première phase* :

| | |
|---|---|
| - Mélange de stéarate de sorbitane et de cocoate de sucrose commercialisé sous la dénomination "ARLATONE 2121" par la société "ICI" | 3,60 g |
| - Cholestérol | 3,60 g |
| - Sel monosodique de l'acide N-stéaroyl glutamique commercialisé sous la dénomination "ACYLGLUTAMATE HS 11" par la société "AJINOMOTO" | 0,80 g |
| - Glycérine | 3,00 g |
| - D-Panthénol | 1,00 g |
| - Mélange d'acides aminés, manitol, saccharose végétal, glycogène marin, acide pyrrolidone carboxylique commercialisé sous la dénomination "HYDROSMYL LS 4513" par les "Laboratoires Sérobiologiques de Nancy" | 0,2 g |
| - Guanosine | 0,01 g |
| - Polyphosphonate commercialisé sous la dénomination de "DEQUEST 2046" par la société "MONSANTO CHEMICAL" | 0,80 g |
| - Hydrolysat lactique commercialisé sous la dénomination de "LACTOLAN LS" par les "Laboratoires Sérobiologiques de Nancy" | 3,00 g |
| - Conservateurs | 0,30 g |
| - Eau déminéralisée | 40 g |

*Deuxième phase* :

| | |
|---|---|
| - Huile de macadamia | 7,50 g |
| - Huile de pépins de raisin | 5,00 g |
| - Huile de silicone volatile | 5,0 g |
| - Filtres antisolaires | 1,00 g |
| - Parfum | 0,20 g |
| - Concentrats naturels de tocophérol | 3,00 g |
| - Mélange d'acides polycarboxyvinyliques commercialisé sous la dénomination de "CARBOPOL 940" par la société "GOODRICH" | 0,42 g |
| - Triéthanolamine qs | pH 6 |
| - Eau déminéralisée qsp | 100 g |

Après une semaine d'application quotidienne, chaque matin, sur le visage et le cou soigneusement nettoyés, on constate une amélioration de l'aspect et de l'éclat de la peau du visage et du cou, qui devient plus lisse, plus ferme, plus hydratée, avec un teint plus éclatant.

**EXEMPLE 23** : Fluide anti-âge pour le visage.

On prépare un fluide ayant la formulation suivante :

*Première phase* :

| | |
|---|---|
| - Polyéthylène glycol 20 distéarate commercialisé sous la dénomination de "GLUCAM E 20 DISTEARATE" par la société "AMERCHOL" | 1,80 g |
| - Cholestérol | 1,80 g |
| - Sel de sodium de l'acide phosphatidique | 0,40 g |
| - Acétate de vitamine E | 0,20 g |
| - Glycérine | 2,00 g |
| - L-Hydroxyproline | 1,00 g |
| - Guanosine | 0,01 g |
| - Solution aqueuse de superoxyde dismutase 5 000 unités/ml commercialisée par la société "PENTAPHARM" | 1,00 g |
| - Conservateurs | 0,30 g |
| - Hydrolysat lactique commercialisé sous la dénomination de "LACTOLAN LS" par les "Laboratoires Sérobiologiques de Nancy" | 5,00 g |
| - Eau déminéralisée | |

*Deuxième phase* :

| | |
|---|---|
| - Huile de pépins de raisin | 4,0 g |
| - Huile de silicone volatile | 2,0 g |
| - Mélange d'acides polycarboxyvinyliques commercialisé sous la dénomination de "CARBOPOL 940" par la société "GOODRICH" | 0,20 g |
| - L-Lysine monohydrate qs | pH 6,5 |
| - Eau déminéralisée qsp | 100 g |

Après une semaine d'application quotidienne, chaque matin, sur le visage soigneusement nettoyé, on constate une amélioration de l'aspect et de l'éclat de la peau du visage, qui devient plus lisse, plus ferme, plus hydratée, avec un teint plus éclatant.

**EXEMPLE 24**: Sérum anti-âge pour le visage.

On prépare un sérum ayant la formulation suivante :

| | |
|---|---|
| - Polyglycéryl-2 stéarate commercialisé sous la dénomination "HOSTACERINE DGS" par la société "HOESCHT" | 1,30 g |
| - Cholestérol | 0,60 g |
| - Sel de sodium de l'acide phosphatidique | 0,10 g |
| - Glycérine | 3,00 g |
| - L-Hydroxyproline | 1,00 g |
| - D-Panthénol | 1,50 g |
| - Guanosine | 0,01 g |
| - Conservateurs | 0,30 g |
| - Polyphosphonate commercialisé sous la dénomination de "DEQUEST 2046" par la société "MONSANTO CHEMICAL" | 0,80 g |
| - Hydrolysat lactique commercialisé sous la dénomination de "LACTOLAN LS" par les "Laboratoires Sérobiologiques de Nancy" | 5,00 g |
| - Solution aqueuse de superoxyde dismutase à 5 000 unités/ml commercialisée par la société "PENTAPHARM" | 1,00 g |
| - Mélange d'acides polycarboxyvinyliques commercialisé sous la dénomination de "CARBOPOL 940" par la société "GOODRICH" | 0,50 g |
| - Triéthanolamine qs | pH 6,5 |
| - Eau déminéralisée qsp | 100 g |

Après une semaine d'application quotidienne, chaque matin, sur le visage soigneusement nettoyé, on constate une amélioration de l'aspect et de l'éclat de la peau du visage, qui devient plus lisse, plus ferme, plus hydratée, avec un teint plus éclatant.

**EXEMPLE 25**: Lait parfumé pour le corps.

On prépare un lait ayant la formulation suivante :

*Première phase :*

| | |
|---|---|
| - Isostéarate de sorbitane commercialisé sous la dénomination "NIKKOL SI 10 R" par la société "NIKKO" | 0,90 g |
| - Sel disodique de l'acide N-stéaroyl glutamique commercialisé sous la dénomination d' "ACYLGLUTAMATE HS 21" par la société "AJINOMOTO" | 0,10 g |
| - Glycérine | 3,00 g |
| - Conservateurs | 0,30 g |
| - Eau déminéralisée | |

*Deuxième phase* :

| | |
|---|---|
| - Huile essentielle de bergamote (exempte de bergaptène) | 5,0 g |
| - Mélange d'acides polycarboxyvinyliques commercialisé sous la dénomination de "CARBOPOL 940" par la société "GOODRICH" | 0,10 g |
| - Triéthanolamine qs | pH 6,5 |
| - Eau déminéralisée qsp | 100 g |

Ce lait appliqué une fois par jour sur le corps sur des sujets à peau ferme donne une amélioration de l'aspect et de l'éclat de la peau, qui devient plus lisse et plus hydratée, de façon appréciable, après 20 jours d'application.

**EXEMPLE 26** : Crème de jour pour le visage.

On prépare une crème ayant la formulation suivante :

*Première phase :*

| | |
|---|---|
| - Distéarate de sucrose commercialisé sous la dénomination "CRODESTA F 160" par la société "CRODA" | 2,20 g |
| - Cholestérol | 1,40 g |
| - Sel de sodium du cholestérol sulfate | 0,40 g |
| - Glycérine | 3,00 g |
| - Eau déminéralisée | |

*Deuxième phase* :

| | |
|---|---|
| - Huile de pépins de raisin | 7,0 g |
| - Huile de tournesol | 5,0 g |
| - Huile de silicone volatile | 4,0 g |
| - Filtres antisolaires | 1,0 g |
| - Mélange d'acides polycarboxyvinyliques commercialisé sous la dénomination de "CARBOPOL 940" par la société "GOODRICH" | 0,42 g |
| - Triéthanolamine qs | pH 6,5 |
| - Eau déminéralisée qsp | 100 g |

Après une semaine d'application quotidienne, chaque matin, sur le visage soigneusement nettoyé, on constate une amélioration de l'aspect et de l'éclat de la peau du visage, qui devient plus lisse, plus ferme, plus hydratée, avec un teint plus éclatant.

**EXEMPLE 27** : Crème de nuit pour le visage destinée à la nutrition des peaux ternes.

On prépare une crème ayant la formulation suivante :

*Première phase :*

| | |
|---|---|
| - Distéarate de saccharose commercialisé sous la dénomination de "GRILLOTEN PSE 141 G" par la société "GRILLO" | 1,80 g |
| - Cholestérol | 1,80 g |
| - Sphingomyéline de lait | 1,80 g |
| - Sel disodique de l'acide N-stéaroyl glutamique commercialisé sous la dénomination "ACYLGLUTAMATE HS 21" par la société "AJINOMOTO" | 0,60 g |
| - Mélange d'acides aminés, manitol, saccharose végétal, glycogène marin, acide pyrrolidone carboxylique non stabilisé commercialisé sous la dénomination "HYDROSMYL LS 4513" par les "Laboratoires Sérobiologiques de Nancy" | 1,00 g |
| - Conservateurs | 0,30 g |
| - Eau déminéralisée | 40 g |

*Deuxième phase* :

| | |
|---|---|
| - Huile de pépins de raisin | 8,00 g |
| - Huile de tournesol | 4,00 g |
| - Huile de silicone volatile | 4,00 g |
| - Parfum | 0,20g |
| - Mélange d'acides polycarboxyvinyliques commercialisé sous la dénomination de "CARBOPOL 940" par la société "GOODRICH" | 0,42 g |
| - Triéthanolamine qs | pH 6,5 |
| - Eau déminéralisée qsp | 100 g |

Après une semaine d'application quotidienne, chaque soir, sur le visage soigneusement nettoyé d'un sujet à peau terne, on constate une amélioration de l'éclat de la peau, qui devient plus lisse, plus ferme, plus hydratée, avec un teint plus éclatant.

**EXEMPLE 28**: Crème de jour anti-âge pour le visage.

On prépare une crème ayant la formulation suivante :

*Première phase :*

| | |
|---|---|
| - Pentastéarate d'hexaglycéryle commercialisé sous la dénomination "HEXAGLYN 5S" par la société "NIKKO" | 2,70 g |
| - Cholestérol | 2,70 g |
| - Sel disodique de l'acide N-stéaroyl glutamique commercialisé sous la dénomination "ACYLGLUTAMATE HS 21" par la société "AJINOMOTO" | 0,60 g |
| - Acétate de vitamine E | 0,60 g |
| - Glycérine | 3,00 g |
| - L-Hydroxyproline | 1,00 g |
| - Guanosine | 0,02 g |
| - Conservateurs | 0,30 g |
| - Polyphosphonate commercialisé sous la dénomination de "DEQUEST 2046" par la société "MONSANTO CHEMICAL" | 0,80 g |
| - Hydrolysat lactique commercialisé sous la dénomination de "LACTOLAN LS" par les "Laboratoires Sérobiologiques de Nancy" | 5,00 g |
| - Eau déminéralisée | 40 g |

*Deuxième phase* :

| | |
|---|---|
| - Huile d'amandes d'abricots | 10,0 g |
| - Huile de silicone volatile | 10,0 g |
| - Filtres antisolaires | 1,00 g |
| - Glycérides de vitamine F | 2,00 g |
| - Mélange d'acides polycarboxyvinyliques commercialisé sous la dénomination de "CARBOPOL 940" par la société "GOODRICH" | 0,50 g |
| - Conservateurs | 0,20 g |
| - Triéthanolamine qs | pH 6,5 |
| - Eau déminéralisée qsp | 100 g |

Après une semaine d'application quotidienne, chaque matin, sur le visage et le cou soigneusement nettoyés, on constate une amélioration de l'aspect et de l'éclat de la peau du visage et du cou, qui devient plus lisse, plus ferme, plus hydratée, avec un teint plus éclatant.

**EXEMPLE 29**: Crème de jour anti-âge pour le visage et le cou destinée au soin des peaux fatiguées.

On prépare une crème ayant la formulation suivante :

*Première phase :*

| | |
|---|---|
| - Décastéarate de décaglycéryle commercialisé sous la dénomination "DECAGLYN 10S" par la société "NIKKO" | 2,70 g |
| - Cholestérol | 2,70 g |
| - Sel disodique de l'acide N-stéaroyl glutamique commercialisé sous la dénomination "ACYLGLUTAMATE HS 21" par la société "AJINOMOTO" | 0,60 g |
| - Acétate de vitamine E | 0,60 g |
| - Glycérine | 3,00 g |
| - L-Hydroxyproline | 1,00 g |
| - Guanosine | 0,02 g |
| - Conservateurs | 0,30 g |
| - Polyphosphonate commercialisé sous la dénomination de "DEQUEST 2046" par la société "MONSANTO CHEMICAL" | 0,80 g |
| - Hydrolysat lactique commercialisé sous la dénomination de "LACTOLAN LS" par les "Laboratoires Sérobiologiques de Nancy" | 5,00 g |
| - Eau déminéralisée | 40 g |

*Deuxième phase* :

| | |
|---|---|
| - Huile d'amandes d'abricots | 10,0 g |
| - Huile de silicone volatile | 10,0 g |
| - Filtres antisolaires | 1,00 g |
| - Glycérides de vitamine F | 2,00 g |
| - Mélange d'acides polycarboxyvinyliques commercialisé sous la dénomination de "CARBOPOL 940" par la société "GOODRICH" | 0,50 g |
| - Conservateurs | 0,20 g |
| - Triéthanolamine qs | pH 6,5 |
| - Eau déminéralisée qsp | 100 g |

Après une semaine d'application quotidienne, chaque matin, sur le visage et le cou soigneusement nettoyés, on constate une amélioration de l'aspect et de l'éclat de la peau du visage et du cou, qui devient plus lisse, plus ferme, plus hydratée, avec un teint plus éclatant.

**EXEMPLE 30** : Crème de jour pour le visage.

On prépare, comme dans l'exemple 8, une crème ayant la formulation suivante :

*Première phase :*

| | |
|---|---|
| Palmitate de sorbitane commercialisé sous la dénomination de "SPAN 40" par la société "ICI" | 3,8 g |
| Cholestérol | 3,8 g |
| Ester α- sulfone de formule 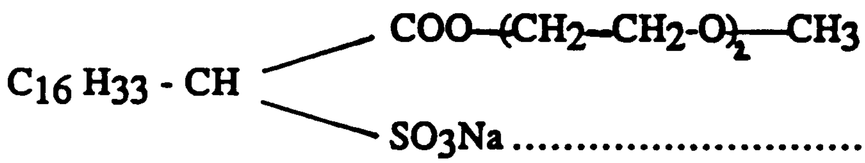 | 0,4 g |
| Eau déminéralisée qsp | 50 g |
| Conservateurs | 0,3 g |
| Glycérine | 5,0 g |

*Deuxième phase :*

| | |
|---|---|
| Huile de macadamia | 16,0 g |
| - Parfum | 0,2 g |
| - Mélange d'acides polycarboxyvinyliques commercialisé sous la dénomination de "CARBOPOL 940" par la société "GOODRICH" | 0,42 g |
| - Triéthanolamine qs | pH 6 |
| - Eau déminéralisée qsp | 100 g |

On mélange, comme indiqué dans l'exemple 8, les deux formulations ci-dessus définies pour obtenir la composition selon l'invention.

Après une semaine d'application quotidienne, chaque matin, sur le visage soigneusement nettoyé, on constate une amélioration de l'aspect de la peau du visage qui devient plus lisse, plus ferme et plus hydratée.

EXEMPLE 31

On prépare un fond de teint ayant la composition suivante :

*Phase A* :

| | |
|---|---|
| - Palmitate de sorbitane commercialisé sous la dénomination commerciale "SPAN 40" par la société "ICI" | 2,85 g |
| - Cholestérol | 2,85 g |
| - Glutamate monosodique commercialisé sous la dénomination commerciale "ACYLGLUTAMATE HS 11" par la société "AJINOMOTO" | 0,3 g |
| - Acétate de vitamine E | 0,3 g |
| - Eau déminéralisée | 35 g |
| - Glycérine | 3 g |
| - Conservateur | 0,1 g |
| - Acide citrique, $1H_2O$ | 0,02 g |

*Phase B*

| | |
|---|---|
| - Néopentanoate d'iso-stéaryle vendu sous la dénomination commerciale "CERAPHIL 375" par la société "VAN DYK" | 5 g |
| - Polyphénylméthylsiloxane vendu sous la dénomination commerciale "DC 556 FLUID COSMETIC" par la société "DOW CORNING" | 20 g |
| - Conservateur | 0,15 g |

*Phase C*

| | |
|---|---|
| - Oxyde de fer jaune enrobé de perfluoroalkylphosphate (95/5) vendu sous la dénomination commerciale "COVAFLUOR" par la société "WACKHERR" | 0,69 g |
| - Oxyde de fer brun enrobé de perfluoroalkylphosphate (95/5) vendu sous la dénomination commerciale "COVAFLUOR" par la société "WACKHERR" | 0,3 g |
| - Oxyde de fer noir enrobé de perfluoroalkylphosphate (95/5) vendu sous la dénomination commerciale "COVAFLUOR" par la société "WACKHERR" | 0,13 g |
| - Dioxyde de titane enrobé de perfluoroalkylphosphate (95/5) vendu sous la dénomination commerciale "COVAFLUOR" par la société "WACKHERR" | 5,88 g |

*Phase D*

| | |
|---|---|
| - Conservateur | 0,3 g |
| - Eau déminéralisée | 1 g |

*Phase E*

| | |
|---|---|
| - Microsphères de silice (diamètre moyen : de 1 à 16 μm) | 2 g |

*Phase F*

| | |
|---|---|
| - Sel disodique de l'acide éthylènediamine tétracétique, $2H_2O$ | 0,05 g |
| - Polymère carboxyvinylique synthétisé dans un mélange acétate d'éthyle/cyclohexane vendu sous la dénomination commerciale "CARBOPOL 950" par la société "GOODRICH" | 0,4 g |
| - Conservateur | 0,1 g |
| - Hydroxyde de sodium | 0,108 g |
| - Eau déminéralisée | 19,472 g |

Le mode opératoire consiste à ajouter les pigments (phase C) dans 1/3 du mélange (dispersion vésiculaire + huiles) (phases A et B), la dispersion étant préalablement réalisée classiquement à l'aide d'un homogénéisateur à haute pression. L'ensemble est mélangé pendant 1 h 30 mn, puis le reste de la dispersion vésiculaire (phase A), les conservateurs (phase D), les charges (phase E) et le gel neutralisé (phase F) sont ajoutés successivement en homogénéisant pendant 5 mn après chaque introduction.

On obtient ainsi un fond de teint de couleur beige naturel. Après une semaine d'application quotidienne, chaque matin, sur le visage soigneusement nettoyé, on constate une amélioration de l'aspect de la peau du visage qui devient plus lisse, plus ferme et plus hydratée.

On a constaté que, dans les crèmes, laits ou sérums des exemples 8 à 31, les vésicules lipidiques sont restées stables après 3 mois de stockage.

**Revendications**

1. Composition formée d'une dispersion aqueuse de vésicules constituées par une membrane de phase lipidique encapsulant une phase aqueuse, la phase lipidique comprenant des lipides amphiphiles non-ioniques et au moins un lipide amphiphile ionique, caractérisée par le fait que :

   - les lipides amphiphiles non-ioniques sont constitués par un mélange d'esters d'au moins un polyol choisi dans le groupe formé par le polyéthylèneglycol comportant de 1 à 60 unités oxyde d'éthylène, le sorbitane, le sorbitane portant 2 à 60 unités d'oxyde d'éthylène, le glycérol portant 2 à 30 unités d'oxyde d'éthylène, les polyglycérols comportant 2 à 15 unités de glycérol, les sucroses, les glucoses portant 2 à 30 unités d'oxyde

d'éthylène, et d'au moins un acide gras comportant une chaîne alkyle en $C_5$-$C_{17}$, saturée ou non saturée, linéaire ou ramifiée, le nombre de chaînes alkyle par groupe polyol étant compris entre 1 et 10 ; et que

- le(s) lipide(s) amphiphile(s) ionique(s) est(sont) choisi(s) dans le groupe formé par ceux conférant à la dispersion un pH compris entre 5,5 et 7,5 ;
- le rapport en poids entre la quantité de lipides amphiphiles non-ioniques et celle de lipide(s) amphiphile(s) ionique(s) dans la phase lipidique étant compris entre 50/1 et 50/25 et le rapport en poids entre la phase lipidique et la phase aqueuse de dispersion étant compris entre 1/1 000 et 300/1 000.

**2.** Composition selon la revendication 1, caractérisée par le fait que le(s) lipide(s) amphiphile(s) ionique(s) est(sont) choisi(s) dans le groupe formé par les lipides anioniques neutralisés, les lipides amphotères et les dérivés alkylsulfoniques.

**3.** Composition selon la revendication 2, caractérisée par le fait que le(s) lipide(s) amphiphile(s) ionique(s) est (sont) choisi(s) dans le groupe formé par :

- les sels alcalins du dicétyl et du dimyristylphosphate,
- les sels alcalins du cholestérol sulfate,
- les sels alcalins du cholestérol phosphate,
- les acylglutamates mono et disodique,
- le sel de sodium de l'acide phosphatidique,
- les phospholipides,
- les dérivés alkylsulfoniques de formule :

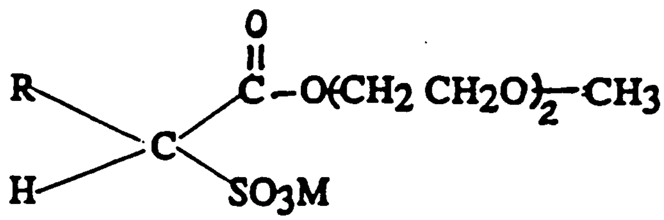

formule dans laquelle R représente les radicaux $C_{16}H_{33}$ et $C_{18}H_{37}$ pris en mélange ou séparément et M est un métal alcalin.

**4.** Composition selon l'une des revendications 1 à 3, caractérisée par le fait que la membrane lipidique des vésicules contient au moins un additif.

**5.** Composition selon la revendication 4, caractérisée par le fait que l'additif est choisi dans le groupe formé par les stérols, les alcools et diols à longue chaîne, les amines à longues chaînes et leurs dérivés ammonium quaternaire.

**6.** Composition selon la revendication 5, caractérisée par le fait que l'additif est le cholestérol.

**7.** Composition selon l'une des revendications 1 à 6, caractérisée par le fait que les vésicules contiennent au moins un actif ayant une activité cosmétique et/ou dermopharmaceutique.

**8.** Composition selon la revendication 7, caractérisée par le fait que les vésicules contiennent au moins un actif hydrosoluble dans la phase aqueuse encapsulée.

**9.** Composition selon la revendication 7, caractérisée par le fait que les vésicules contiennent au moins un actif liposoluble dans la phase lipidique constituant leur membrane.

**10.** Composition selon la revendication 9, caractérisée par le fait que l'actif liposoluble est choisi dans le groupe formé par les sphingomyélines, les glycocéramides et les céramides naturels ou synthétiques.

**11.** Composition selon les revendications 6 et 10 prises en combinaison, caractérisée par le fait que la phase lipidique constituant la membrane contient un mélange de cholestérol et de céramide(s) naturel(s) ou de synthèse.

12. Composition selon la revendication 7, caractérisée par le fait que les vésicules contiennent un actif amphiphile qui se répartit entre la phase lipidique et la phase aqueuse encapsulée.

13. Composition selon l'une des revendications 1 à 12, caractérisée par le fait que la phase aqueuse de dispersion contient une dispersion de gouttelettes d'un liquide non miscible à l'eau.

14. Composition selon l'une des revendications 1 à 13, caractérisée par le fait que la phase aqueuse de dispersion contient au moins un adjuvant pris dans le groupe formé par les gélifiants, les conservateurs, les colorants, les pigments, les charges, les opacifiants et les parfums.

15. Composition selon la revendication 14, comportant, comme adjuvant, des pigments, caractérisée par le fait que lesdits pigments sont des pigments enrobés par un produit choisi dans le groupe formé par les silicones, les composés fluorés ou les amino-acides.

16. Composition selon la revendication 15, comportant comme adjuvant des pigments enrobés par des composés fluorés, caractérisée par le fait que lesdits composés fluorés sont choisis dans le groupe formé par les perfluoroalkylphosphates et le polytétrafluoroéthylène.

17. Composition selon l'une des revendications 14 à 16, comportant, comme adjuvant, des pigments, caractérisée par le fait que lesdits pigments sont des oxydes de fer ou de titane.

18. Composition selon la revendication 14, comportant, comme adjuvant, des charges, caractérisée par le fait que lesdites charges sont choisies dans le groupe formé par le talc, le mica, la poudre d'amidon, la poudre de nylon et la poudre de silice.

## Claims

1. Composition composed of an aqueous dispersion of vesicles consisting of a membrane of lipid phase encapsulating an aqueous phase, the lipid phase comprising nonionic amphiphilic lipids and at least one ionic amphiphilic lipid, characterized in that:

- the nonionic amphiphilic lipids consist of a mixture of esters of at least one polyol chosen from the group composed of polyethylene glycol containing from 1 to 60 ethylene oxide units, sorbitan, sorbitan bearing 2 to 60 ethylene oxide units, glycerol bearing 2 to 30 ethylene oxide units, polyglycerols containing 2 to 15 glycerol units, sucroses, and glucoses bearing 2 to 30 ethylene oxide units, and of at least one fatty acid containing a saturated or unsaturated, linear or branched $C_5$-$C_{17}$ alkyl chain, the number of alkyl chains per polyol group being between 1 and 10; and in that
- the ionic amphiphilic lipid or lipids is/are chosen from the group composed of those which impart a pH of between 5.5 and 7.5 to the dispersion;
- the weight ratio of the quantity of nonionic amphiphilic lipids to that of ionic amphiphilic lipid or lipids in the lipid phase being between 50:1 and 50:25 and the weight ratio of the lipid phase to the aqueous dispersion phase being between 1:1,000 and 300:1,000.

2. Composition according to Claim 1, characterized in that the ionic amphiphilic lipid or lipids is/are chosen from the group composed of neutralized anionic lipids, amphoteric lipids and alkylsulphonic derivatives.

3. Composition according to Claim 2, characterized in that the ionic amphiphilic lipid or lipids is/are chosen from the group composed of:

- the alkali metal salts of dicetyl and dimyristyl phosphates,
- the alkali metal salts of cholesterol sulphate,
- the alkali metal salts of cholesterol phosphate,
- mono- and disodium acylglutamates,
- phosphatidic acid sodium salt,
- phospholipids,

- the alkylsulphonic derivates of formula:

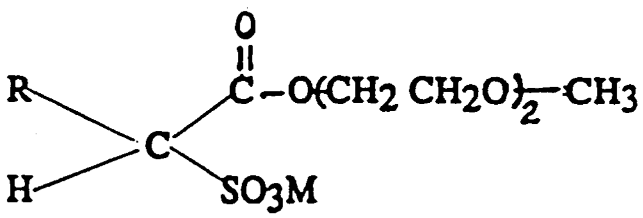

in which formula R represents $C_{16}H_{33}$ and $C_{18}H_{37}$ radicals taken mixed or separately and M is an alkali metal.

**4.** Composition according to one of Claims 1 to 3, characterized in that the lipid membrane of the vesicles contains at least one additive.

**5.** Composition according to Claim 4, characterized in that the additive is chosen from the group composed of sterols, long-chain alcohols and diols, and long-chain amines and their quaternary ammonium derivatives.

**6.** Composition according to Claim 5, characterized in that the additive is cholesterol.

**7.** Composition according to one of Claims 1 to 6, characterized in that the vesicles contain at least one active agent having cosmetic and/or dermopharmaceutical activity.

**8.** Composition according to Claim 7, characterized in that the vesicles contain at least one water-soluble active agent in the encapsulated aqueous phase.

**9.** Composition according to Claim 7, characterized in that the vesicles contain at least one fat-soluble active agent in the lipid phase constituting their membrane.

**10.** Composition according to Claim 9, characterized in that the fat-soluble active agent is chosen from the group composed of sphingomyelins, glycoceramides and natural or synthetic ceramides.

**11.** Composition according to Claims 6 and 10 taken in combination, characterized in that the lipid phase constituting the membrane contains a mixture of cholesterol and natural or synthetic ceramide(s).

**12.** Composition according to Claim 7, characterized in that the vesicles contain an amphiphilic active agent which distributes between the lipid phase and the encapsulated aqueous phase.

**13.** Composition according to one of Claims 1 to 12, characterized in that the aqueous dispersion phase contains a dispersion of droplets of a water-immiscible liquid.

**14.** Composition according to one of Claims 1 to 13, characterized in that the aqueous dispersion phase contains at least one adjuvant selected from the group composed of gelling agents, preservatives, colorants, pigments, fillers, opacifiers and perfumes.

**15.** Composition according to Claim 14, containing pigments as an adjuvant, characterized in that the said pigments are pigments coated with a product chosen from the group composed of silicones, fluorinated compounds or amino acids.

**16.** Composition according to Claim 15, containing pigments coated with fluorinated compounds as an adjuvant, characterized in that the said fluorinated compounds are chosen from the group composed of perfluoroalkyl phosphates and polytetrafluoroethylene.

**17.** Composition according to one of Claims 14 to 16, containing pigments as an adjuvant, characterized in that the said pigments are oxides of iron or of titanium.

18. Composition according to Claim 14, containing fillers as an adjuvant, characterized in that the said fillers are chosen from the group composed of talc, mica, starch powder, nylon powder and silica powder.

## Patentansprüche

1. Zusammensetzung umfassend eine wässrige Dispersion von Vesikeln, bestehend aus einer Membran aus einer Lipidphase, die eine wässrige Phase einschließt, wobei die Lipidphase amphiphile nichtionische Lipide und mindestens ein amphiphiles ionisches Lipid enhält, dadurch gekennzeichnet, daß:

   - die amphiphilen nichtionischen Lipide gebildet werden aus einer Mischung von Estern aus mindestens einem Polyol, das aus der Gruppe der folgenden Verbindungen gewählt ist: Polyethylenglykol, das 1 bis 60 Ethylenoxideinheiten trägt, Sorbitan, Sorbitan, das 2 bis 60 Ethylenoxideinheiten trägt, Glycerin, das 2 bis 30 Ethylenoxideinheiten trägt, Polyglycerinen mit 2 bis 15 Glycerineinheiten, Saccharosen, Glukosen, die 2 bis 30 Ethylenoxideinheiten tragen,
   und aus mindestens einer Fettsäure, mit einer gesättigten oder ungesättigten, linearen oder verzweigten $C_5$-$C_{17}$-Alkylkette, wobei die Anzahl Alkylketten pro Polyolgruppe 1 bis 10 beträgt; und daß

   - das (die) amphiphile(n) ionische(n) Lipid(e) aus der Gruppe der Verbindungen gewählt ist (sind), die der Dispersion einen pH von 5,5 bis 7,5 verleihen;

   - das Gewichtsverhältnis von der Menge der amphiphilen nichtionischen Lipide zu jener des (der) amphiphilen ionischen Lipids (Lipide) in der Lipidphase 50:1 bis 50:25 beträgt und das Gewichtsverhältnis von Lipidphase zur wässrigen Phase der Dispersion 1:1 000 bis 300:1 000 beträgt.

2. Zusammensetzung nach Anspruch 1, dadurch gekennzeichnet, daß das (die) amphiphile(n), ionische(n) Lipide(n) ausgewählt ist (sind) unter neutralisierten anionischen Lipiden, amphoteren Lipiden und Alkylsulfonderivaten.

3. Zusammensetzung nach Anspruch 2, dadurch gekennzeichnet, daß das (die) amphiphile(n) ionische(n) Lipid(e) ausgewählt ist (sind) unter den:

   Alkalisalzen von Dicetyl- und Dimyristylphosphat,
   Alkalisalzen von Cholesterinsulfat,
   Alkalisalzen von Cholesterinphosphat,
   Mono- und Dinatriumacylglutamaten,
   Natriumsalzen der Phosphorsäure,
   Phospholipiden,
   Alkylsulfonderivaten der Formel:

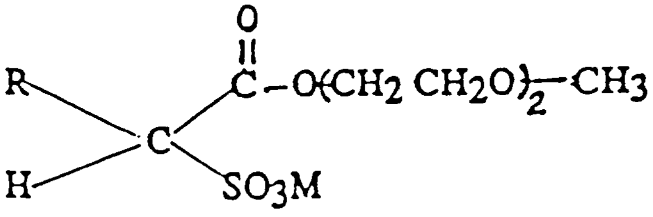

   wobei R für einen $C_{16}H_{33}$-Rest, einen $C_{18}H_{37}$-Rest oder deren Mischungen steht und M ein Alkalimetall ist.

4. Zusammensetzung nach einem der Ansprüche 1 bis 3, dadurch gekennzeichnet, daß die Lipidmembran der Vesikeln mindestens ein Additiv enthält.

5. Zusammensetzung nach Anspruch 4, dadurch gekennzeichnet, daß das Additiv ausgewählt ist unter: Sterinen, langkettigen Alkoholen und Diolen, langkettigen Aminen und deren quaternären Ammoniumderivaten.

6. Zusammensetzung nach Anspruch 5, dadurch gekennzeichnet, daß das Additiv Cholesterin ist.

7. Zusammensetzung nach einem der Ansprüche 1 bis 6, dadurch gekennzeichnet, daß die Vesikel mindestens einen Wirkstoff mit einer kosmetischen und/oder dermatopharmazeutischen Wirkung enthalten.

8. Zusammensetzung nach Anspruch 7, dadurch gekennzeichnet, daß die Vesikel mindestens einen wasserlöslichen Wirkstoff in der eingeschlossenen wässrigen Phase enthalten.

9. Zusammensetzung nach Anspruch 7, dadurch gekennzeichnet, daß die Vesikel mindestens einen fettlöslichen Wirkstoff in der Fettphase, die ihre Membran bilden, enthalten.

10. Zusammensetzung nach Anspruch 9, dadurch gekennzeichnet, daß der fettlösliche Wirkstoff aus der Gruppe der folgenden Verbindungen gewählt wird: Sphingomyelinen, Glycoceramiden und natürlichen oder synthetischen Ceramiden.

11. Zusammensetzung nach den Ansprüchen 6 und 10, dadurch gekennzeichnet, daß die die Membran bildende Fettphase eine Mischung aus Cholesterin und einem oder mehreren natürlichen oder synthetischen ceramiden enthält.

12. Zusammensetzung nach Anspruch 7, dadurch gekennzeichnet, daß die Vesikel einen amphiphilen Wirkstoff, der sich auf die Fettphase und die eingeschlossene wässrige Phase verteilt, enthalten.

13. Zusammensetzung nach einem der Ansprüche 1 bis 12, dadurch gekennzeichnet, daß die wässrige Phase der Dispersion eine tröpfchenförmige Dispersion einer mit Wasser nicht mischbaren Flüssigkeit enthält.

14. Zusammensetzung nach einem der Ansprüche 1 bis 13, dadurch gekennzeichnet, daß die wässrige Phase der Dispersion mindestens ein Adjuvans, ausgewählt unter Gelbildnern, Konservierungsstoffen, Farbstoffen, Pigmenten, Füllstoffen, Trübungsmitteln und Parfums enthält.

15. Zusammensetzung nach Anspruch 14, umfassend als Adjuvans Pigmente, dadurch gekennzeichnet, daß die Pigmente mit einem Mittel, ausgewählt unter Silikonen, fluorierten Verbindungen oder Aminosäuren, umhüllt sind.

16. Zusammensetzung nach Anspruch 15, umfassend als Adjuvans mit fluorierten Verbindungen umhüllte Pigmente, dadurch gekennzeichnet, daß die fluorierten Verbindungen unter Perfluoralkylphosphaten und Polytetrafluorethylen ausgewählt sind.

17. Zusammensetzung nach einem der Ansprüche 14 bis 16, umfassend als Adjuvans Pigmente, dadurch gekennzeichnet, daß die Pigmente Oxide von Eisen oder Titan sind.

18. Zusammensetzung nach Anspruch 14, umfassend als Adjuvans Füllstoffe, dadurch gekennzeichnet, daß die Füllstoffe unter Talkum, Glimmer, Stärkepulver, Nylonpulver und Siliciumdioxidpulver ausgewählt sind.